# EUROPEAN PATENT APPLICATION

(11) **EP 4 837 917 A2**
(43) Date of publication of application: **11.11.2026**
(21) Application number: 26168393.2
(22) Date of filing: 27.03.2026
(51) Int. Cl.: C07H 21/02

(54) **CAPPING ANALOGS FOR SELF-REPLICATING MRNA AND USES THEREOF**

(30) Priority: 06.05.2025 CN 202510570674
(71) Applicant: Beijing Youcare Kechuang Pharmaceutical Technology Co., Ltd., Daxing District, Beijing 100176 (CN)
(72) Inventor: ZHANG, Honglei, Beijing, 100176 (CN); SONG, Gengshen, Beijing, 100176 (CN); MA, Yuqing, Beijing, 100176 (CN); YU, Fei, Beijing, 100176 (CN); JIN, Lijie, Beijing, 100176 (CN); LIU, Lijie, Beijing, 100176 (CN); MA, Teng, Beijing, 100176 (CN); MA, Jingxuan, Beijing, 100176 (CN); DONG, Kai, Beijing, 100176 (CN); ZHANG, Man, Beijing, 100176 (CN); WANG, Huanyu, Beijing, 100176 (CN)
(74) Representative: WSL Patentanwälte Partnerschaft mbB

(57) **Abstract**

The present disclosure provides a capping analog for self-replicating mRNA and uses thereof, wherein the capping analog for self-replicating mRNA is capable of enhancing the stability and/or translation efficiency of the self-replicating mRNA.

## Description

### TECHNICAL FIELD

The present disclosure relates to the technical field of chemical and biological engineering, specifically to a capping analog for self-replicating mRNA and uses thereof.

### BACKGROUND OF THE INVENTION

The cap structure is an essential element for RNA to perform its biological functions *in vivo.* Its presence enables RNA to evade cellular innate immune responses, facilitate efficient *in vivo* protein translation, and serves as a primary prerequisite for stimulating the body's response. The chemical essence of the cap structure is a special structure located at the 5' end of RNA formed by modifications during RNA transcription, namely the m7GPPPN structure, also known as the methylguanosine cap. It is formed through the combined catalytic action of RNA triphosphatase, guanylyltransferase, RNA (guanine-N7) methyltransferase, and RNA (nucleoside-2') methyltransferase.

Self-replicating RNA (srRNA), also commonly referred to as self-amplifying RNA (saRNA), is a special type of RNA molecule that possesses the ability to replicate itself. Compared to traditional mRNA, the key distinction of self-replicating mRNA lies in its ability to replicate itself using its own RNA sequence as a template. This self-replicating property enables higher protein expression with lower vaccine doses, thereby enhancing vaccine efficacy.

Similar to traditional mRNA, self-replicating mRNA also requires a cap structure to enhance its stability and translation efficiency within cells. Self-replicating mRNA is typically designed based on the genomes of positive-sense RNA viruses, such as Venezuelan equine encephalitis virus (VEEV), Semliki Forest virus (SFV), and Sindbis virus (SIN). The genomes of these positive-sense RNA viruses initiate with a 5'-AU sequence, and the m7GpppApU-type capping analog can match well with this initiation sequence, thereby better simulating the natural infection process of the virus.

Due to differences in sequence length and cap structure types, the structural modification methods used for m7GpppApG capping analogs in traditional mRNA preparation processes may not be applicable to the development of self-replicating mRNA capping analogs. Therefore, providing a capping analog suitable for self-replicating mRNA is crucial.

### SUMMARY OF THE INVENTION

The capping analog for self-replicating mRNA described in the present disclosure can improve the stability and/or translation efficiency of self-replicating mRNA.

The present application provides a compound represented by formula (I), a stereoisomer thereof or a pharmaceutically acceptable salt thereof: 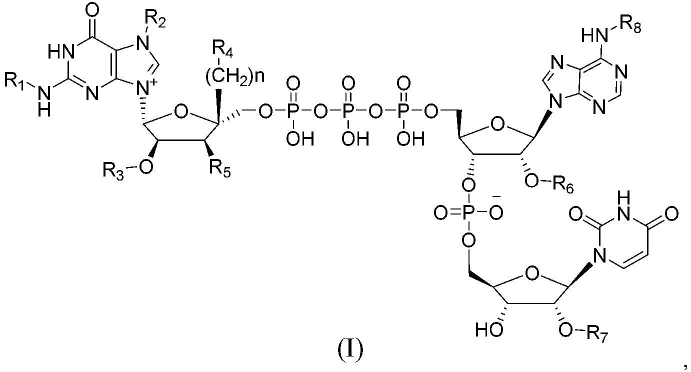
where n is 0, 1, 2 or 3;
R₅ is -OR₅ₐ, -SR_{5b}, -NR_{5c}R_{5d}, -C(O)R_{5c}, 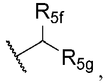 -NHC(O)-R₅ₕ, -N(CH₃)C(O)-R₅ᵢ, -C(O)NR₅ⱼR₅ₖ, -C(O)O-R₅ₗ, -OC(O)-R₅ₘ or -S(O)₂-R₅ₙ;
R₄ₐ, R₅ₐ, R_{5b}, R_{5c}, R_{5d}, R_{5c}, R₅ₕ, R₅ᵢ, R₅ⱼ, R₅ₖ, R₅ₗ, R₅ₘ and R₅ₙ are independently -H, C₁-C₃ alkyl or halogenated C₁-C₃ alkyl;
R_{5f} and R_{5g} are independently -H, halogen, C₁-C₃ alkyl, -OC₁-C₃ alkyl, halogenated C₁-C₃ alkyl or -NR₅₋₁C(O)R₅₋₂;
R₅₋₁ and R₅₋₂ are independently -H or C₁-C₃ alkyl;
R₁, R₂, R₃, R₆ and R₇ are each independently -H, C₁-C₆ alkyl, C₂₋C₆ alkenyl, C₂-C₆ alkynyl, C₃-C₆ cycloalkyl or benzyl;
R₄ is -OR₄ₐ, halogen or -H; and
R₈ is -H or C₁-C₃ alkyl.

In some embodiments, in the compound represented by formula (I), a stereoisomer thereof or a pharmaceutically acceptable salt thereof, the definition of certain groups is as described below and the definition of the remaining groups is as described in any other embodiment (hereinafter referred to as "in some embodiments"):
n is 0 or 1.

In some embodiments, R₄ is H, -OC₁-C₃ alkyl or halogen.

In some embodiments, R₄ is H or halogen.

In some embodiments, R₄ is H, -F or -OCH₃.

In some embodiments, R₅ is -OH, 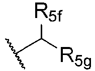 or -C(O)NR₅ⱼR₅ₖ; R_{5f} and R_{5g} are independently halogen, C₁-C₃ alkyl, -OC₁-C₃ alkyl or -NR₅₋₁C(O)R₅₋₂; R₅ⱼ and R₅ₖ are independently C₁-C₃ alkyl; R₅₋₁ is H; and R₅₋₂ is C₁-C₃ alkyl.

In some embodiments, R₅ is -OH, 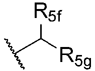 or -C(O)NR₅ⱼR₅ₖ; R_{5f} is C₁-C₃ alkyl or halogen; R_{5g} is halogen, C₁-C₃ alkyl or -OC₁-C₃ alkyl; and R₅ⱼ and R₅ₖ are independently C₁-C₃ alkyl.

In some embodiments, R₅ is -OH,

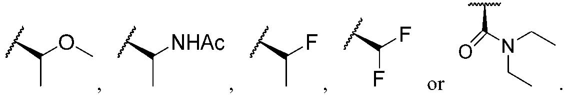

In some embodiments, R₅ is -OH,

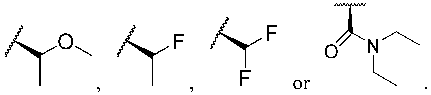

In some embodiments, R₁ is -H.

In some embodiments, R₂ is C₁-C₃ alkyl.

In some embodiments, R₂ is -CH₃.

In some embodiments, R₃ is H.

In some embodiments, R₆ is C₁-C₃ alkyl.

In some embodiments, R₆ is -CH₃.

In some embodiments, R₇ is -H.

In some embodiments, R₈ is -H.

In some embodiments, n is 0 or 1; R₄ is -H or halogen;
R₅ is -OH, 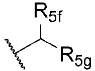 or -C(O)NR₅ⱼR₅ₖ; R_{5f} and R_{5g} are independently halogen, C₁-C₃ alkyl or -OC₁-C₃ alkyl; R₅ⱼ and R₅ₖ are independently C₁-C₃ alkyl;
R₁ is -H; R₂ is C₁-C₃ alkyl; R₃ is -H;
R₆ is C₁-C₃ alkyl; R₇ is -H; and
R₈ is -H.

In some embodiments, the pharmaceutically acceptable salts of the compound represented by formula (I) have any of the following structures: 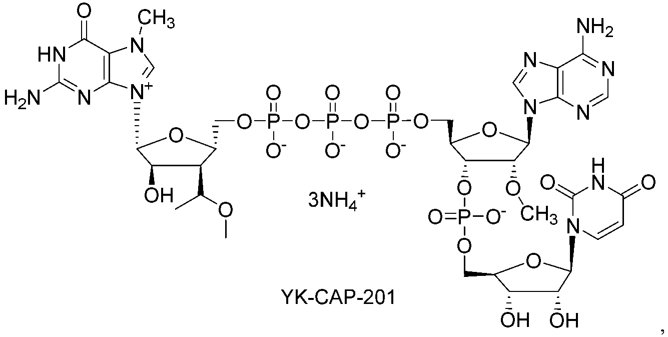 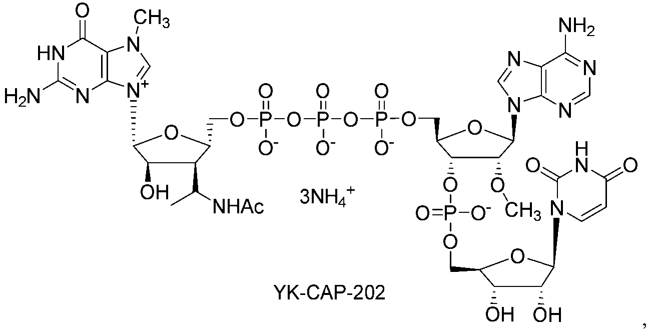 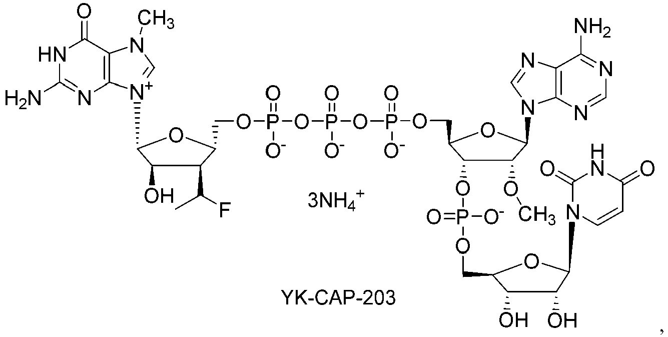 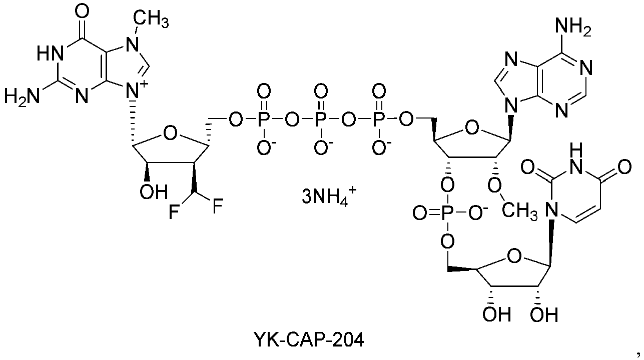 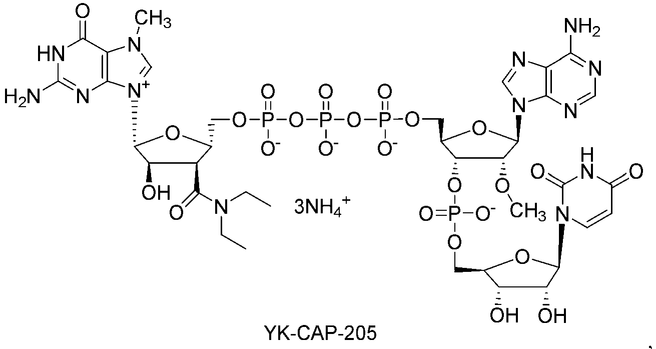 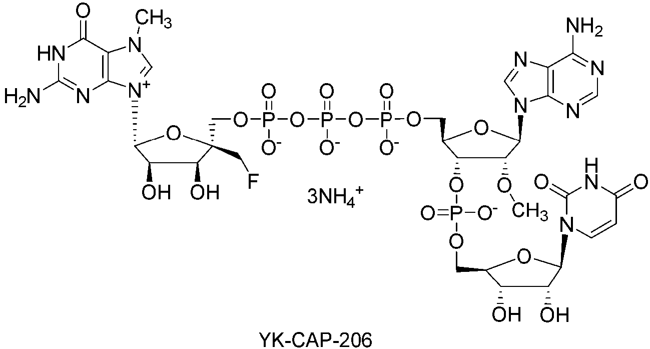 or

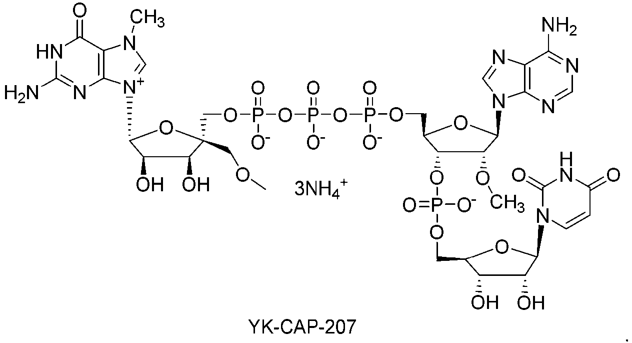

The present application provides a use of the compound represented by formula (I), a stereoisomer thereof or a pharmaceutically acceptable salt thereof, in the preparation of an *in vitro* co-transcription mRNA capping reagent.

The present application also provides an RNA molecule comprising the compound represented by formula (I), a stereoisomer thereof or a pharmaceutically acceptable salt thereof as described above as a cap structure or cap structure fragment.

The present application also provides a pharmaceutical composition comprising the RNA molecule as described above.

In some embodiments, the pharmaceutical composition further comprises at least one delivery agent.

In some embodiments, the at least one delivery agent comprises at least one cationic lipid.

In some embodiments, the cationic lipid is any one or a combination of at least two of the following substances:
(i) a compound represented by formula (II), a stereoisomer thereof, a N-oxide thereof, a solvate thereof or a pharmaceutically acceptable salt thereof, wherein G₁ is C₁₋₆ alkylene; G₂ is C₂₋₈ alkylene; G₃ is C₁₋₃ alkylene; L₁ is C₆₋₁₅ linear alkyl; and L₂ is C₁₂₋₂₅ branched alkyl; 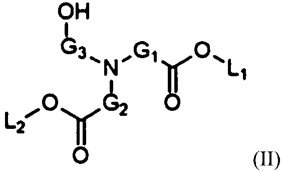
(ii) a compound represented by formula (III), a stereoisomer thereof, a N-oxide thereof, a solvate thereof or a pharmaceutically acceptable salt thereof, wherein G₁ is C₂₋₈ alkylene; G₂ is C_{2∼8} alkylene; L₁ is -C(O)O- or -OC(O)-; L₂ is -C(O)O- or -OC(O)-; R₁ is C₆₋₂₅ linear or branched alkyl; R₂ is C₆₋₂₅ linear or branched alkyl; G₃ is HO(CH₂)₂- or HO(CH₂)₃-; G₄ is HO(CH₂)₂- or HO(CH₂)₃-; and L is -(CH₂)₂-, -(CH₂)₃- or -(CH₂)₄-; 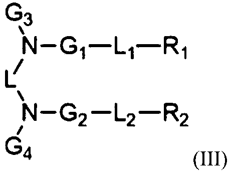
(iii) a compound represented by formula (IV), a stereoisomer thereof, a N-oxide thereof, a solvate thereof or a pharmaceutically acceptable salt thereof, wherein G₁ is C₁₋₆ alkylene; G₂ is C₂₋₈ alkylene; R₁ is C₆₋₂₀ linear or branched alkyl; R₂ is C₁₂₋₂₅ branched alkyl; and G₃ is HO(CH₂)₂N(CH₃)(CH₂)₂-, HO(CH₂)₂N(CH₂CH₃)(CH₂)₂-, (HO(CH₂)₂)₂N(CH₂)₂-, CH₃O(CH₂)₂N(CH₃)(CH₂)₂-, (CH₃)₂N(CH₂)₃SC(O)O(CH₂)₂-, (CH₃)₂N(CH₂)₃SC(O)-, CH₃NH(CH₂)₂N(CH₃)(CH₂)₂- or CH₃CH₂NH(CH₂)₂-; 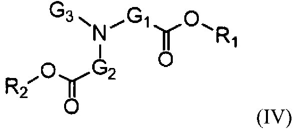
(iv) a compound represented by formula (V), a stereoisomer thereof, a N-oxide thereof, a solvate thereof or a pharmaceutically acceptable salt thereof, wherein G₁ is C₁₋₈ alkylene; G₂ is C₂₋₈ alkylene; R₁ is C₆₋₂₅ linear or branched alkyl; R₂ is C₁₂₋₂₅ linear or branched alkyl; and G₃ is HO(CH₂)₂N(R₃)CH₂CH(OH)CH₂-, where R₃ is -CH₃, -CH₂CH₃ or -CH₂CH₂OH; 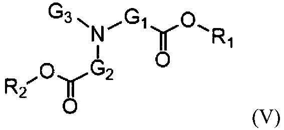
(v) a compound represented by formula (VI), a stereoisomer thereof, a N-oxide thereof, a solvate thereof or a pharmaceutically acceptable salt thereof, wherein G¹ and G² are each independently C₆-C₁₀ alkylene; G³ is C₁-C₁₂ alkylene; R¹ and R² are each independently C₆-C₂₄ alkyl or C₆-C₂₄ alkenyl; R³ is OR⁵, N(R⁵)₂, -C(= O)OR⁴, -OC(=O)R⁴ or -NR⁵C(=O)R⁴; R⁴ is C₁-C₁₂ hydrocarbyl; and R⁵ is H or C₁-C₆ hydrocarbyl; 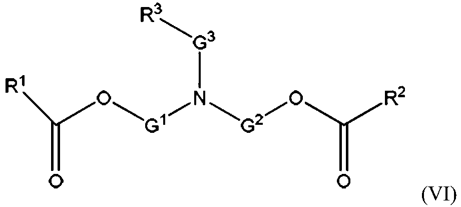
(vi) a compound represented by formula (VII), a stereoisomer thereof, a N-oxide thereof, a solvate thereof or a pharmaceutically acceptable salt thereof, wherein R₄ is -(CH₂)ₙQ; Q is -OR, -OH, -O(CH₂)ₙN(R)₂, -OC(O)R, -CX₃, -CN, -N(R)C(O)R, -N(H)C(O)R, -N(R)S(O)₂R, -N(H)S(O)₂R, -N(R)C(O)N(R)₂, -N(H)C(O)N(R)₂, -N(H)C(O)N(H)(R), -N(R)C(S)N(R)₂, -N(H)C(S)N(R)₂, -N(H)C(S)N(H)(R), -N(R)S(O)₂R or heterocyclyl; n is 1, 2 or 3; R is independently C₁₋₈ alkyl; and X is independently H or C₁₋₈ alkyl; 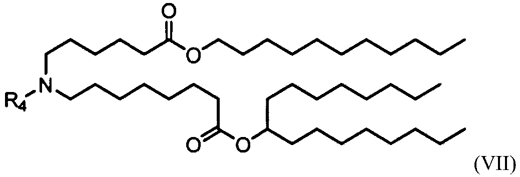
(vii) a compound represented by formula (VIII), a stereoisomer thereof, a N-oxide thereof, a solvate thereof or a pharmaceutically acceptable salt thereof,

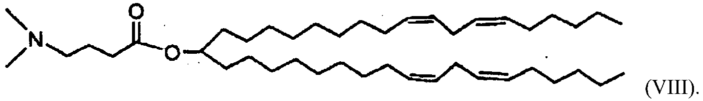

In some embodiments, the cationic lipid is selected from the group consisting of YK-009, YK-201, YK-407, YK-305, ALC-0315, SM-102 and DLIN-MC3-DMA, or a combination of at least two thereof:

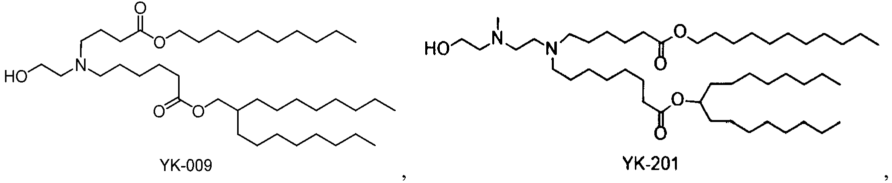

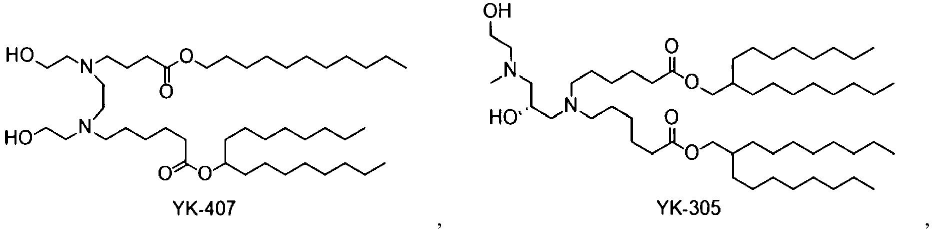

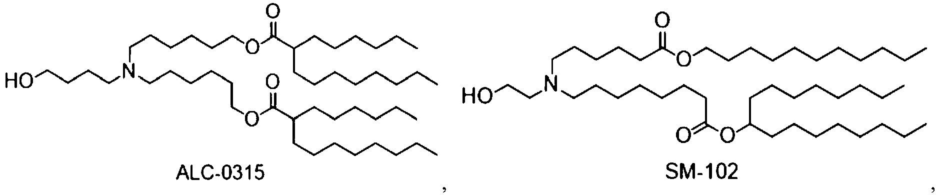

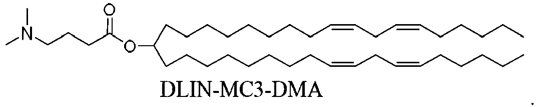

In some embodiments, the cationic lipid is the YK-009.

In some embodiments, the at least one delivery agent further comprises at least one neutral lipid.

In some embodiments, the neutral lipid is selected from the group consisting of phosphatidylcholine, phosphatidylethanolamine, sphingomyelin, ceramide and sterol, or a combination of at least two thereof.

In some embodiments, the neutral lipid is selected from the group consisting of 1,2-dilinoleoyl-sn-glycero-3-phosphocholine, 1,2-dimyristoyl-sn-glycero-phosphocholine, 1,2-dioleoyl-sn-glycero-3-phosphocholine, 1,2-dipalmitoyl-sn-glycero-3-phosphocholine, 1,2-distearoyl-sn-glycero-3-phosphocholine (DSPC), 1,2-diundecanoyl-sn-glycero-phosphocholine, 1-palmitoyl-2-oleoyl-sn-glycero-3-phosphocholine, 1,2-di-O-octadecenyl-sn-glycero-3-phosphocholine, 1-oleoyl-2-cholesterylhemisuccinoyl-sn-glycerol-3-phosphocholine, 1-hexadecyl-sn-glycero-3-phosphocholine, 1,2-dilinolenoyl-sn-glycero-3-phosphocholine, 1,2-diarachidonoyl-sn-glycero-3-phosphocholine, 1,2-bis(docosahexaenoyl)-sn-glycero-3-phosphocholine, 1,2-dioleoyl-sn-glycerol-3-phosphoethanolamine (DOPE), 1,2-diphytanoyl-sn-glycero-3-phosphoethanolamine, 1,2-distearoyl-sn-glycero-3-phosphoethanolamine, 1,2-dilinoleoyl-sn-glycero-3-phosphoethanolamine, 1,2-dilinolenoyl-sn-glycero-3-phosphoethanolamine, 1,2-diarachidonoyl-sn-glycero-3-phosphoethanolamine, 1,2-bis(docosahexaenoyl)-sn-glycero-3-phosphoethanolamine, 1,2-dioleoyl-sn-glycero-3-phosphate-rac-(1-glycerol) sodium salt, dipalmitoyl phosphatidylglycerol, palmitoyl oleoyl phosphatidylethanolamine, distearoyl-phosphatidyl-ethanolamine, dipalmitoyl phosphatidyl ethanolamine, dimyristoyl phosphoethanolamine, 1-stearoyl-2-oleoyl-stearoyl-ethanolamine, 1-stearoyl-2-oleoyl-phosphatidylcholine, sphingomyelin, phosphatidylcholine, phosphatidylethanolamine, phosphatidylserine, phosphatidylinositol, phosphatidic acid, palmitoyl oleoyl phosphatidylcholine, lysophosphatidylcholine and lysophosphatidylethanolamine, or a combination of at least two thereof.

In some embodiments, the neutral lipid is 1,2-distearoyl-sn-glycero-3-phosphocholine and/or 1,2-dioleoyl-sn-glycero-3-phosphoethanolamine.

In some embodiments, the at least one delivery agent further comprises a structural lipid.

In some embodiments, the structural lipid is selected from the group consisting of cholesterol, nonsterol, sitosterol, ergosterol, campesterol, stigmasterol, brassicasterol, tomatine, ursolic acid, α-tocopherol and corticosteroid, or a combination of at least two thereof.

In some embodiments, the structural lipid is cholesterol.

In some embodiments, the at least one delivery agent further comprises a polymer conjugated lipid.

In some embodiments, the polymer conjugated lipid is selected from the group consisting of 1,2-distearoyl-sn-glycero-3-phosphoethanolamine-N-[methoxy(polyethylene glycol)-2000, 1,2-dimyristoyl-sn-glycero-3-methoxypolyethylene glycol 2000 (DMG-PEG2000) and methoxypoly(ethylene glycol) ditetradecylacetamide, or a combination of at least two thereof.

In some embodiments, the polymer conjugated lipid is 1,2-dimyristoyl-sn-glycero-3-methoxypolyethylene glycol 2000.

In some embodiments, the at least one delivery agent comprises YK-009, 1,2-distearoyl-sn-glycero-3-phosphocholine, cholesterol and 1,2-dimyristoyl-sn-glycero-3-methoxypolyethylene glycol 2000; and the YK-009 is

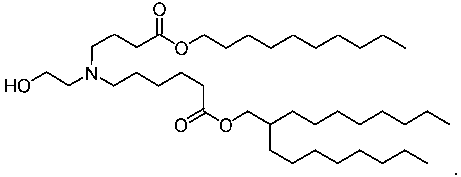

In some embodiments, a molar ratio of the YK-009, 1,2-distearoyl-sn-glycero-3-phosphocholine, cholesterol and 1,2-dimyristoyl-sn-glycero-3-methoxypolyethylene glycol 2000 is preferably 49: 10: 39.5: 1.5.

In some embodiments, the pharmaceutical composition further comprises one or at least two cell-penetrating peptides (CPPs).

The present application provides a method for synthesizing mRNA molecules for non-diagnostic and non-therapeutic purpose with respect to diseases, wherein the method comprises the following steps: co-incubating the compound represented by formula (I), a stereoisomer thereof or a pharmaceutically acceptable salt thereof as described above with a polynucleotide template, and performing transcription of the polynucleotide template.

The present application provides a capping mRNA transcription response system for non-diagnostic and non-therapeutic purpose with respect to diseases, comprising: (1) the compound represented by formula (I), a stereoisomer thereof or a pharmaceutically acceptable salt thereof as described above; and (2) a polynucleotide template, NTPs, and RNA polymerase.

The present application provides a kit, comprising: (1) the compound represented by formula (I), a stereoisomer thereof or a pharmaceutically acceptable salt thereof as described above; and (2) nucleoside triphosphate molecules and RNA polymerase.

In some embodiments, the kit further comprises any one or a combination of at least two of the group consisting of an RNase inhibitor, an inorganic pyrophosphatase, Mg²⁺, a crowding agent, and a buffer.

The present application provides a method for enhancing RNA stability, wherein the method comprises incorporating the compound represented by formula (I), a stereoisomer thereof or a pharmaceutically acceptable salt thereof as described above into the RNA.

The present application provides a method for introducing RNA into cells, wherein the method comprises contacting the cells with the RNA molecule as described above or the pharmaceutical composition as described above.

The present application provides use of the RNA molecule as described above or the pharmaceutical composition as described above in the preparation of a vaccine.

### Definition of Terms

All publications and patents referenced herein are hereby incorporated by reference in their entirety into the present disclosure. If any uses or terminology employed in publications and patents incorporated by reference conflict with those used in the present disclosure, the uses and terminology of the present disclosure shall prevail.

The chapter titles used in the present application are intended solely for organizational purposes and should not be interpreted as limiting the subject matter.

Unless otherwise specified, all technical and scientific terms used in the present application have the usual meanings in the field to which the claimed subject matter pertains. If multiple definitions exist for a term, the definition provided herein shall prevail.

Unless specified in the examples or otherwise indicated, all numerical values for quantitative properties such as dosage described herein shall be understood to be modified by the term "approximately" in all instances. It should also be understood that any numerical range cited herein is intended to encompass all subranges within that range and any combination of the respective endpoints of the range or subranges.

Additionally, when referring to numbers or numerical ranges, the term "approximately" indicates that the mentioned numbers or numerical ranges are approximations within typical tolerances ranges in the art, within experimental variability, or within statistical experimental error. Consequently, such numbers or numerical ranges may vary, for example, between 1% and 15% of the stated numbers or numerical ranges. For example, "approximately" can be understood as about two standard deviations from the mean. When "approximately" precedes a series of numbers or numerical ranges, it should be understood that "approximately" can modify each number within the series or ranges.

Unless otherwise defined, all terms used herein, including technical and scientific terms, shall have the same meaning as commonly understood by a person skilled in the art to which the present disclosure pertains. Terms, such as those defined in common dictionaries, shall be interpreted as having meanings consistent with their context in the relevant technology, and shall not be interpreted in an idealized or overly formal sense, unless explicitly defined as such in the present disclosure.

In the present disclosure, the 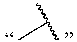 in a structural fragment indicates that the structural fragment is connected to the remainder of the molecule via that bond. For example, 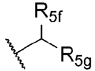 indicates that it is connected to the remainder of the molecule via

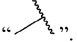

As used in the present disclosure, the term "C₁-C₃" refers to a group in which the number of carbon atoms in the main chain has any integer value in the range of 1 to 3, for example, 1, 2, or 3 carbon atoms. The term "C₆₋₁₅" refers to a group in which the number of carbon atoms has any integer value in the range of 6 to 15, such as 6, 7, 8, 9, 10, 11, 12, 13, 14, or 15 carbon atoms. Similarly, the limitation of the range of carbon atoms indicates that the number of carbon atoms in the specified group may be any integer value within the defined range.

As used in the present disclosure, the term "alkyl" refers to a saturated aliphatic hydrocarbyl group with a linear or branched chain; its non-limiting examples include methyl, ethyl, propyl, isopropyl, and the like.

In the present application, the term "alkoxy" refers to the group R^{Y}-O-, and the definition of R^{Y} is the same as the term "alkyl". Alkoxy includes but is not limited to: methoxy, ethoxy, n-propoxy, isopropoxy, etc.

The term "alkenyl" refers to a linear or branched hydrocarbyl group with at least one double bond, consisting only of carbon and hydrogen atoms, having, for example, 2 to 6, such as 2, 3, 4, 5, or 6 carbon atoms, and connected to the remainder of the molecule through a single bond. Alkenyl includes but is not limited to vinyl, 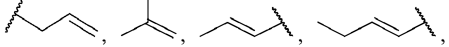 etc.

The term "alkynyl" refers to a linear or branched hydrocarbyl group with at least one triple bond, consisting only of carbon and hydrogen atoms, having, for example, 2 to 6, such as 2, 3, 4, 5, or 6 carbon atoms, and connected to the remainder of the molecule through a single bond. Alkynyl includes but is not limited to ethynyl, 1-propynyl, but-1-ynyl, but-2-ynyl, etc.

The term "halogen" refers to fluorine, chlorine, bromine, or iodine.

The term "cycloalkyl" refers to a saturated monocyclic ring group consisting only of carbon atoms with a specified number of carbon atoms (such as C₃-C₆). Cycloalkyl includes but is not limited to cyclopropyl, cyclobutyl, cyclopentyl, cyclohexyl, etc.

As used in the present disclosure, the term "salt" refers to the corresponding salt for the modification of nucleoside compounds (or nucleotide compounds) in the present disclosure that can be conveniently or appropriately prepared, purified, and/or processed, such as pharmaceutically acceptable salts. Unless otherwise specified, references to specific compounds in the present disclosure also include their salt forms.

As used in the present disclosure, "capping analog" refers to a structure formed at the 5' end of mature RNA following post-transcriptional modification in eukaryotes, namely the m7GPPPN structure, also known as the methylguanosine cap. This structure can have the functions of preventing RNA degradation at the 5' end, assisting RNA transcription products to pass through selective pores of the nuclear membrane and enter the cytoplasm, enhancing translation, and helping to complete the entire splicing process.

The terms "including", "containing", or "comprising" and the like, as used in the present disclosure mean that the elements preceding such terms encompass the elements listed thereafter and their equivalents, without excluding elements not specifically described. The terms "containing" or "including (comprising)" used herein may be open-ended, semi-closed, or closed. In other words, the term also encompasses "consisting essentially of..." or "consisting of...".

The term "pharmaceutically acceptable" in the present disclosure refers to a compound or composition that is chemically and/or toxicologically compatible with other components of the formulation and/or with humans or mammals using it for the prevention or treatment of diseases or conditions.

The term "polynucleotide template" in the present disclosure refers to a polynucleotide chain serving as the basis for synthesizing other nucleic acid molecules.

The present disclosure also includes salts of the compounds described herein, particularly pharmaceutically acceptable salts. Compounds of the present disclosure having functional groups that are sufficiently acidic or sufficiently basic can react with a variety of bases or acids to form salts. Alternatively, compounds bearing inherent charges, such as those containing quaternary nitrogen, can form salts with suitable counterions, such as halide ions like bromide ions, chloride ions, or fluoride ions, particularly bromide ions.

The pharmaceutically acceptable salts in the present disclosure may be, for example, acid addition salts of the compounds in the present disclosure that are sufficiently basic and contain nitrogen atoms in the chain or ring of the structural compound represented by formula (I), such as acid addition salts formed with the following inorganic acids: such as hydrochloric acid, hydrobromic acid, hydroiodic acid, sulfuric acid, phosphoric acid, or nitric acid; or acid addition salts formed with the following organic acids: such as formic acid, acetic acid, acetoacetic acid, pyruvic acid, trifluoroacetic acid, propionic acid, butyric acid, hexanoic acid, heptanoic acid, undecanoic acid, lauric acid, benzoic acid, salicylic acid, 2-(4-hydroxybenzoyl) benzoic acid, camphoric acid, cinnamic acid, cyclopentylpropionic acid, 3-hydroxy-2-naphthoic acid, nicotinic acid, pamoic acid, pectinic acid, peroxosulfuric acid, 3-phenylpropionic acid, picric acid, pivalic acid, 2-hydroxyethanesulfonic acid, itaconic acid, aminosulfonic acid, trifluoromethanesulfonic acid, dodecyl sulfuric acid, ethanesulfonic acid, benzenesulfonic acid, p-toluenesulfonic acid, methanesulfonic acid, 2-naphthalenesulfonic acid, naphthalene disulfonic acid, camphorsulfonic acid, citric acid, tartaric acid, stearic acid, lactic acid, oxalic acid, malonic acid, succinic acid, malic acid, adipic acid, alginic acid, maleic acid, fumaric acid, D-gluconic acid, mandelic acid, ascorbic acid, glucoheptonic acid, glycerophosphoric acid, aspartic acid, sulfosalicylic acid or thiocyanic acid.

In addition, another suitable pharmaceutically acceptable salt of the compound in the present disclosure with sufficient acidity is an alkali metal salt such as a sodium salt or a potassium salt, an alkaline earth metal salt such as a calcium salt or a magnesium salt, an ammonium salt such as a salt formed with NH₃ or aqueous ammonia, or a salt formed with an organic base that provides physiologically acceptable cations, such as a salt formed with the following substances: triethylamine, N-methylglucosamine, dimethylglucosamine, ethylglucosamine, lysine, dicyclohexylamine, 1,6-hexanediamine, ethanolamine, glucosamine, sarcosine, serine, tri(hydroxymethyl)aminomethane, aminopropanediol, 1-amino-2,3,4-butanetriol. In addition, alkaline nitrogen-containing groups can be quaternized using the following reagents: lower alkyl halides such as methyl, ethyl, propyl, and butyl chlorides, bromides, and iodides; dialkyl sulfate esters, such as dimethyl sulfate, diethyl sulfate, dibutyl sulfate, and dipentyl sulfate; long chain halides such as decyl, laurel, nutmeg, and stearyl chlorides, bromides, and iodides; and arylalkyl halides such as benzyl and phenethyl bromides.

Those skilled in the art will also recognize that the acid addition salts of the compounds of formula (I) in the present disclosure may be prepared by reacting the compounds with suitable inorganic or organic acids using any of the known methods. Alternatively, the base addition salts of the acidic compounds in the present disclosure can be prepared by reacting them with appropriate bases using various known methods.

The present disclosure includes all possible salts of the compound of formula (I) in the present disclosure, which may be a single salt or any mixture of the salts in any proportion.

Certain compounds of the present disclosure may exist in the form of one or more stereoisomers. Stereoisomers include geometric isomers, diastereoisomers, and enantiomers. Therefore, the compounds of formula (I) in the present disclosure also include racemic mixtures, single stereoisomers, and optically active mixtures. Those skilled in the art should understand that one stereoisomer may exhibit superior efficacy and/or reduced side effects compared to other stereoisomers. Single stereoisomer and optically active mixtures can be obtained through methods such as chiral pool synthesis, chiral catalysis, and chiral resolution. Racemates may undergo chiral resolution through chromatographic resolution or chemical resolution methods. For example, chiral acids such as chiral tartaric acid and chiral malic acid can be used as resolving agents to form salts with the compounds of the present disclosure. Separation can be achieved by exploiting differences in the physicochemical properties of the products, such as solubility.

In the present disclosure, when the name of a compound differs from its structural formula, the structural formula shall prevail.

The specification of the present disclosure should be interpreted as being consistent with the rules and principles of chemical bonds. In some cases, hydrogen atoms may be removed to accommodate substituents at a given position.

It should be understood that the terms "compounds of the present disclosure" and "compounds" as used herein may refer, depending on the context, to: compounds of formula (I), solvates thereof, pharmaceutically acceptable salts thereof, stereoisomers thereof, or their mixtures.

Based on common knowledge in the field, the above preferred conditions may be combined arbitrarily to obtain various preferred embodiments of the present disclosure.

Unless otherwise specified, all reagents and raw materials used in the present disclosure are commercially available.

The positive and progressive effect of the present disclosure lies in the fact that the compound represented by formula (I) provided in the present disclosure serves as a mRNA capping analog for self-replication and has at least one of the following advantages:
(1) the capping analogs for self-replicating mRNA of the present application significantly improve both mRNA *in vitro* transcription yield and integrity; and (2) the capping analogs for self-replicating mRNA of the present application significantly enhance mRNA translation efficiency.

### DESCRIPTION OF DRAWINGS

In order to more clearly illustrate the technical solutions in the detailed description of the present disclosure or the prior art, the accompanying drawings to be used in the detailed description or the prior art will be briefly introduced below, and it will be obvious that the accompanying drawings in the following description are some of the embodiments of the present disclosure and are not intended to limit the scope of the present disclosure. For those skilled in the art, various modifications made within the spirit and scope of the present disclosure may also be considered to fall within the scope intended by the accompanying drawings of the present disclosure.
Figure 1 shows the *in vitro* transcription yield results of mRNA using YK-CAP-201, YK-CAP-202, YK-CAP-203, YK-CAP-204, YK-CAP-205, YK-CAP-206, YK-CAP-207, and N-7114 as capping analogs.
Figure 2 shows the results of mRNA transcription integrity initiated by YK-CAP-201, YK-CAP-202, YK-CAP-203, YK-CAP-204, YK-CAP-205, YK-CAP-206, YK-CAP-207, and N-7114 as capping analogs.
Figure 3 shows the capping rate results for mRNA transcription initiated by YK-CAP-201, YK-CAP-202, YK-CAP-203, YK-CAP-204, YK-CAP-205, YK-CAP-206, YK-CAP-207, and N-7114 as capping analogs.
Figure 4 shows the translation efficiency results of capped mRNA using YK-CAP-201, YK-CAP-202, YK-CAP-203, YK-CAP-204, YK-CAP-205, YK-CAP-206, YK-CAP-207, and N-7114 as capping analogs.
Figure 5 shows the *in vivo* expression results in mice of capped mRNA using YK-CAP-201, YK-CAP-202, YK-CAP-203, YK-CAP-204, YK-CAP-205, YK-CAP-206, YK-CAP-207, and N-7114 as capping analogs.

### DETAILED DESCRIPTION

The following examples provide further description of the present disclosure, but the present disclosure is not limited to these examples. The implementation conditions used in the examples can be further adjusted according to different requirements for specific use, and the unspecified implementation conditions are conventional conditions in this field. In the specific examples of the present disclosure, all raw materials used are commercially available. Unless otherwise specified, all temperatures are given in degrees Celsius. The technical features described in the various embodiments of the present disclosure may be combined with one another provided that they do not conflict with each other.

The following abbreviations respectively represent the following reagents:
TEA: Triethylamine; DMF: N,N-Dimethylformamide; PySSPy: 2,2'-Dithiodipyridine; PPh₃: Triphenylphosphine; TEAB: Triethylammonium hydrogen carbonate; EDTA: Ethylenediaminetetraacetic acid; DSPC: 1,2-Distearoyl-sn-glycero-3-phosphocholine; DMG-PEG 2000: 1,2-Dimyristoyl-sn-glycero-3-methoxypolyethylene glycol 2000.

### Example 1: synthesis of a capping analog for self-replicating mRNA

### 1. Synthesis of intermediate INT-I

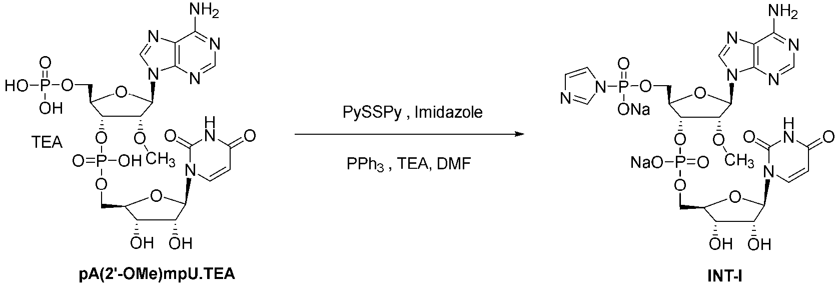

pA(2'-OMe)mpU•TEA (3.10 g, 4.64 mmol), imidazole (1.58 g, 23.22 mmol), 2,2'-dithiodipyridine (2.04 g, 9.28 mmol), triethylamine (0.47 g, 4.64 mmol), and triphenylphosphine (2.43 g, 9.28 mmol) were dissolved in 31 ml of ultradry N,N-dimethylformamide to obtain a mixture. The mixture was reacted at 25 °C for 4 h under nitrogen protection. After the reaction was complete, the reaction mixture was slowly added to a pre-cooled solution of sodium iodide (5.08 g, 33.89 mmol) in acetone (150 ml). Crystallization was carried out at 25 °C for 30 min, followed by centrifugation and freeze-drying to obtain a white solid, INT-I (2.50 g, 3.28 mmol, 75.0% yield). C₂₃H₂₉N₉O₁₄P₂, MS(ES): m/z (M-H)⁻ 716.1.

### 2. Synthesis of YK-CAP-201

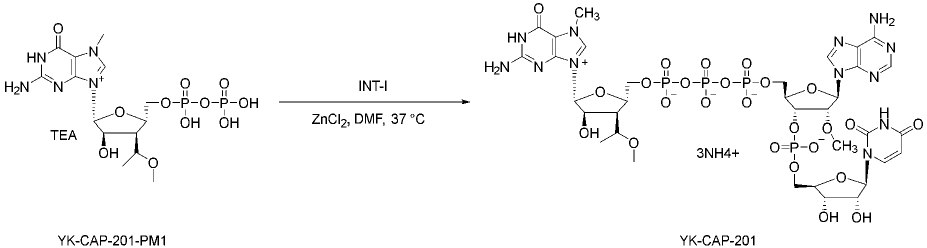

YK-CAP-201-PM1 (synthesized according to the synthesis route of YK-CAP-107-PM11 in patent CN118389495B) (130 mg, 0.26 mmol) and INT-I (513 mg, 0.72 mmol) were dissolved in DMF, ZnCl₂ was added to obtain a mixture. The mixture was stirred at 37 °C for 72 hours under N₂ protection. After the reaction was complete, the reaction mixture was poured into an aqueous EDTA solution and stirred for 20 minutes. The pH was adjusted to 6.5 using aqueous ammonia, followed by purification via gel column (elution with water and 1 M TEAB in a ratio of 3: 1). The target product was collected, concentrated, and lyophilized. Further purification was carried out by high performance preparative liquid chromatograph, resulting in the final product YK-CAP-201 (54 mg, 47 µmol, 18.1%), C₃₄H₄₉N₁₂O₂₅P₄, MS(ES): m/z (M-H)⁻ 1147.2. ¹H NMR (400 MHz, D₂O) δ 9.09 (s, 1H), 8.43 (s, 1H), 8.17 (s, 1H), 7.41 (d, *J =* 2.4 Hz, 1H), 6.03 (d, *J* = 4.4 Hz, 1H), 5.71 (d, *J =* 5.6 Hz, 1H), 5.47 (d, *J =* 2.4 Hz, 1H), 4.90 - 4.83 (m, 1H), 4.67 (t, *J* = 5.4 Hz, 2H), 4.52 (d, *J =* 5.2 Hz, 1H), 4.47 - 4.43 (m, 2H), 4.42 - 4.38 (m, 2H), 4.34 (t, *J =* 4.8 Hz, 1H), 4.31 - 4.23 (m, 2H), 4.21 - 4.15 (m, 1H), 4.14 - 4.11 (m, 2H), 4.10 - 4.06 (m, 1H), 3.93 (s, 3H), 3.66 - 3.55 (m, 1H), 3.42 (s, 3H), 3.25 (s, 3H), 2.33 - 2.24 (m, 1H), 1.12 (d, *J =* 6.0 Hz, 3H). ³¹P NMR (162 MHz, D₂O) δ -0.88, -11.46, -11.60, -22.76 (4P).

### 3. Synthesis of YK-CAP-202

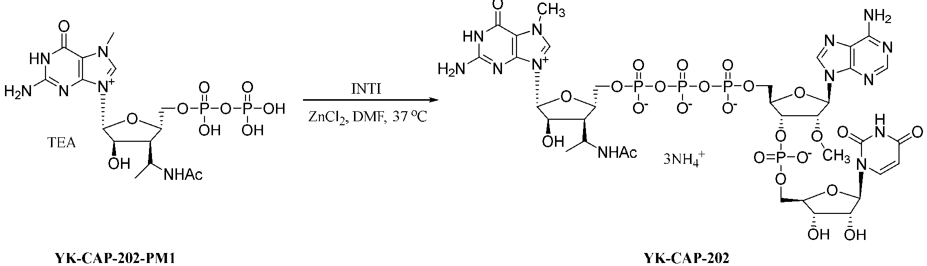

YK-CAP-202-PM1 (synthesized according to the synthesis route of YK-CAP-108-PM10 in patent CN118389495B) (100 mg, 0.19 mmol) and INT-I (179 mg, 0.25 mmol) were used as raw materials, the final product YK-CAP-202 (20 mg, 17 µmol, 9.0%) was obtained according to the synthesis route of YK-CAP-201. C₃₅H₄₉N₁₃O₂₅P₄, MS(ES): m/z (M-H)⁻ 1174.1. ¹H NMR (400 MHz, D₂O) δ 9.06 (s, 1H), 8.36 (s, 1H), 8.12 (s, 1H), 7.36 (d, *J* = 2.4 Hz, 1H), 6.01 (d, *J* = 4.4 Hz, 1H), 5.68 (d, *J =* 5.6 Hz, 1H), 5.44 (d, *J =* 2.4 Hz, 1H), 4.90 - 4.80 (m, 1H), 4.64 (t, *J =* 5.4 Hz, 2H), 4.48 (d, *J =* 5.2 Hz, 1H), 4.44 - 4.40 (m, 2H), 4.36 - 4.32 (m, 2H), 4.29 (t, *J =* 4.8 Hz, 1H), 4.26 - 4.19 (m, 2H), 4.18 - 4.11 (m, 1H), 4.10 - 4.08 (m, 2H), 4.06 - 4.01 (m, 1H), 3.88 (s, 3H), 3.63 - 3.51 (m, 1H), 3.40 (s, 3H), 2.29 - 2.20 (m, 1H), 1.90 (s, 3H), 1.08 (d, *J =* 6.0 Hz, 3H). ³¹P NMR (162 MHz, D₂O) δ -0.89, -11.47, -11.63, -22.77 (4P).

### 4. Synthesis of YK-CAP-203

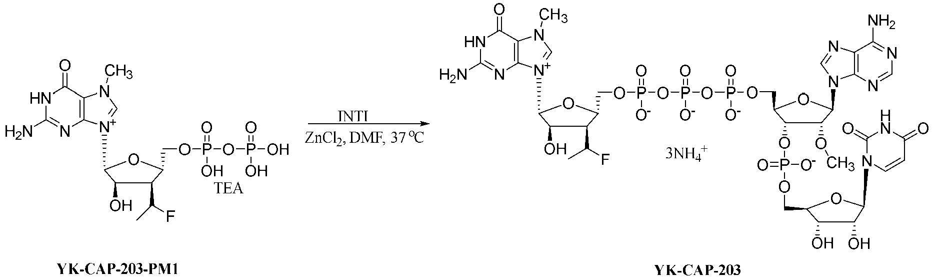

YK-CAP-203-PM1 (synthesized according to the synthesis route of YK-CAP-109-PM8 in patent CN118389495B) (100 mg, 0.17 mmol) and INT-I (179 mg, 0.25 mmol) were used as raw materials, the final product YK-CAP-203 (15 mg, 13 µmol, 6.5%) was obtained according to the synthesis route of YK-CAP-201. C₃₃H₄₅FN₁₂O₂₄P₄, MS(ES): m/z (M-H)⁻ 1135.1. ¹HNMR (400 MHz, D₂O) δ 9.08 (s, 1H), 8.42 (s, 1H), 8.15 (s, 1H), 7.40 (d, *J =* 2.4 Hz, 1H), 6.17 (d, *J* = 4.4 Hz, 1H), 5.65 (d, *J =* 2.6 Hz, 1H), 5.47 (d, *J = 2.4* Hz, 1H), 5.32 - 5.21 (m, 1H), 4.62 - 4.42 (m, 3H), 4.36 - 4.33 (m, 4H), 4.22 - 4.15 (m, 3H), 4.11 (s, 2H), 4.03 (s, 1H), 3.91 - 3.82 (m, 3H), 3.11 - 3.15 (m, 3H), 3.11 (s, 2H), 2.31 - 2.22 (m, 1H), 1.52 (d, J = 6.1 Hz, 3H). ³¹PNMR (163MHz, D₂O) δ -0.89, -11.21, -11.74, -22.12 (4P).

### 5. Synthesis of YK-CAP-204

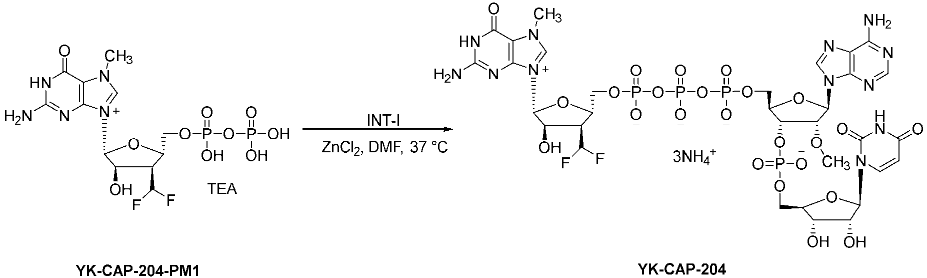

YK-CAP-204-PM1 (synthesized according to the synthesis route of YK-CAP-110-PM8 in patent CN118389495B) (180 mg, 0.31 mmol) and INT-I (470 mg, 0.62 mmol) were used as raw materials, the final product YK-CAP-204 (71 mg, 49 µmol, 15.8%) was obtained according to the synthesis route of YK-CAP-201. C₃₂H₃₉F₂N₁₂O₂₄P₄, MS(ES): m/z (M-H)⁻ 1138.62. ¹H NMR (400 MHz, D₂O) δ 9.01 (s, 1H), 8.33 (s, 1H), 8.06 (s, 1H), 7.35 (d, *J =* 2.4 Hz, 1H), 6.30 - 5.99 (m, 1H), 5.96 (d, *J =* 5.2 Hz, 1H), 5.75 - 5.71 (m, 2H), 5.40 (d, *J =* 2.4 Hz, 1H), 4.90 - 4.82 (m, 1H), 4.81 - 4.76 (m, 1H), 4.68 - 4.61 (m, 4H), 4.45 - 4.31 (m, 6H), 4.30 - 4.21 (m, 3H), 4.20 - 4.10 (m, 4H), 4.07 - 4.00 (m, 2H), 3.93 (s, 4H), 3.38 (s, 4H), 2.97 - 2.85 (m, 1H). ³¹P NMR (162 MHz, D₂O) δ -0.88, -11.50, -11.64, -22.76 (4P).

### 6. Synthesis of YK-CAP-205

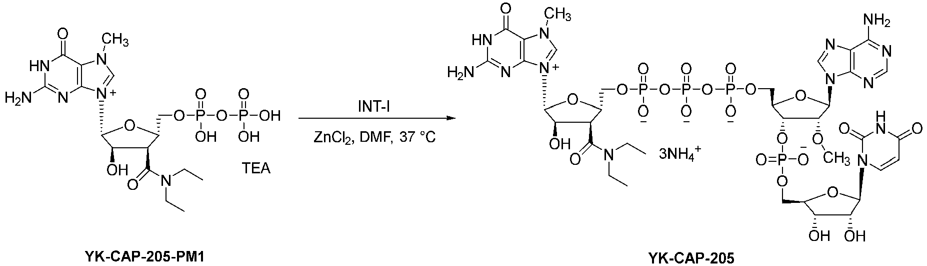

YK-CAP-205-PM1 (synthesized according to the synthesis route of YK-CAP-111-PM10 in patent CN118389495B) (120 mg, 0.22 mmol) and INT-I (477 mg, 0.67 mmol) were used as raw materials, the final product YK-CAP-205 (17 mg, 14 µmol, 6.5%) was obtained according to the synthesis route of YK-CAP-201. C₃₆H₅₂N₁₃O₂₅P₄, MS(ES): m/z (M-H)⁻ 1188.2. ¹H NMR (400 MHz, D₂O) δ 9.11 (s, 1H), 8.82 (s, 1H), 8.60 (s, 1H), 7.51 (d, *J =* 2.4 Hz, 1H), 6.09 (d, *J* = 4.8 Hz, 1H), 5.82 (d, *J =* 3.6 Hz, 2H), 5.53 (d, *J =* 2.4 Hz, 1H), 4.90 - 4.84 (m, 2H), 4.82 - 4.78 (m, 1H), 4.62 (t, *J =* 4.4 Hz, 1H), 4.52 - 4.36 (m, 5H), 4.34 - 4.09 (m, 6H), 4.08 - 4.00 (m, 1H), 3.95 (s, 3H), 3.72 (t, *J =* 6.4 Hz, 1H), 3.43 (s, 3H), 3.39 - 3.21 (m, 5H), 1.05 (dt, *J =* 21.2, 7.2 Hz, 7H). ³¹P NMR (162 MHz, D₂O) δ -0.92, 11.48, -11.73, -22.57 (4P).

### 7. Synthesis of YK-CAP-206

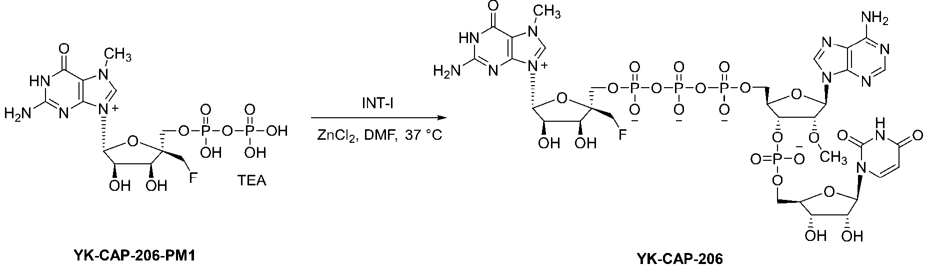

YK-CAP-206-PM1 (synthesized according to the synthesis route of YK-CAP-118-PM8 in patent CN118389495B) (200 mg, 0.41 mmol) and INT-I (954 mg, 1.34 mmol) were used as raw materials, the final product YK-CAP-206 (44 mg, 39 µmol, 9.4%) was obtained according to the synthesis route of YK-CAP-201. C₃₄H₄₄N₁₂O₂₅P₄, MS(ES): m/z (M-H)⁻ 1137.2. ¹H NMR (400 MHz, D₂O) δ 9.01 (s, 1H), 8.36 (s, 1H), 8.09 (s, 1H), 7.35 (d, *J =* 2.4 Hz, 1H), 5.95 (d, *J* = 5.2 Hz, 1H), 5.86 (d, *J =* 5.2 Hz, 1H), 5.73 (d, *J =* 5.6 Hz, 1H), 5.40 (d, *J* = 2.4 Hz, 1H), 4.87 - 4.81 (m, 1H), 4.68 - 4.64 (m, 2H), 4.53 (d, *J =* 5.6 Hz, 1H), 4.47 - 4.35 (m, 4H), 4.33 (t, *J =* 5.2 Hz, 2H), 4.28 - 4.21 (m, 2H), 4.14 - 4.09 (m, 3H), 3.95 (s, 3H), 3.69 (d, *J =* 10.8 Hz, 1H), 3.59 (d, *J =* 10.8 Hz, 1H), 3.35 (s, 3H). ³¹P NMR (162 MHz, D₂O) δ -0.90, -11.41, -11.48, -22.68 (4P).

### 8. Synthesis of YK-CAP-207

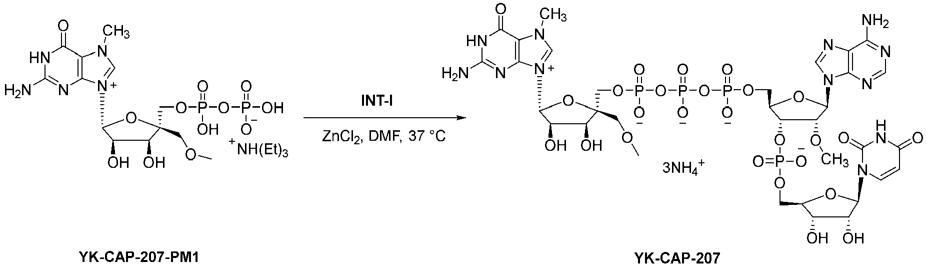

YK-CAP-207-PM1 (synthesized according to the synthesis route of YK-CAP-117-PM8 in patent CN118389495B) (180 mg, 0.35 mmol) and INT-I (514 mg, 0.71 mmol) were used as raw materials, the final product YK-CAP-207 (21 mg, 18 µmol, 5.0%) was obtained according to the synthesis route of YK-CAP-201. C₃₃H₄₇N₁₂O₂₆P₄, MS(ES): m/z (M-H)⁻ 1149.18. ¹H NMR (400 MHz, D₂O) δ 9.01 (s, 1H), 8.36 (s, 1H), 8.09 (s, 1H), 7.35 (d, *J =* 2.4 Hz, 1H), 5.95 (d, *J* = 5.2 Hz, 1H), 5.86 (d, *J =* 5.2 Hz, 1H), 5.73 (d, *J =* 5.6 Hz, 1H), 5.40 (d, *J =* 2.4 Hz, 1H), 4.87 - 4.81 (m, 1H), 4.68 - 4.64 (m, 2H), 4.53 (d, *J =* 5.6 Hz, 1H), 4.44 - 4.39 (m, 2H), 4.33 (t, *J =* 5.2 Hz, 2H), 4.28 - 4.21 (m, 2H), 4.19 - 4.15 (m, 2H), 4.14 - 4.09 (m, 3H), 3.95 (s, 3H), 3.69 (d, *J =* 10.8 Hz, 1H), 3.59 (d, *J =* 10.8 Hz, 1H), 3.38 (s, 3H), 3.35 (s, 3H). ³¹P NMR (162 MHz, D₂O) δ -0.92, -11.44, *-*11.55*,* -22.74 (4P).

### Example 2: mRNA in vitro transcription yield and capping rate

### I. Structural differences in capping analogs

**Table 1 Structures of capping analogs**

| Name | Structure | Note |
|---|---|---|
| YK-CAP-201 | 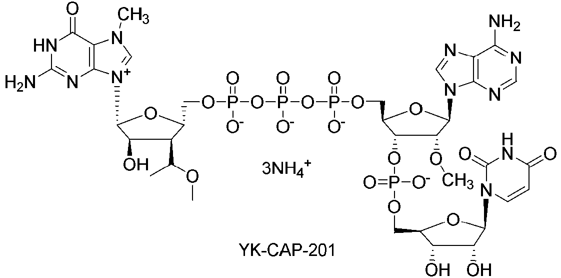 | The structure designed in the present application, synthesized in Example 1 |
| YK-CAP-202 | 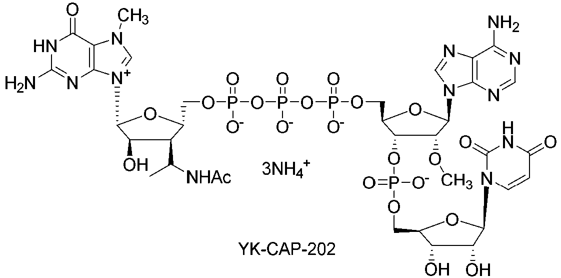 | The structure designed in the present application, synthesized in Example 1 |
| YK-CAP-203 | 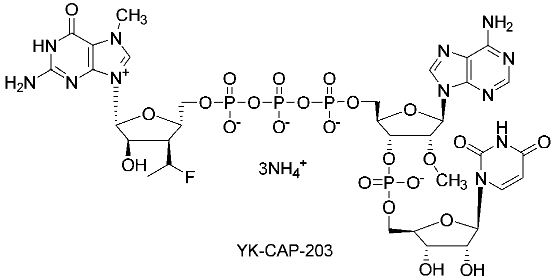 | The structure designed in the present application, synthesized in Example 1 |
| YK-CAP-204 | 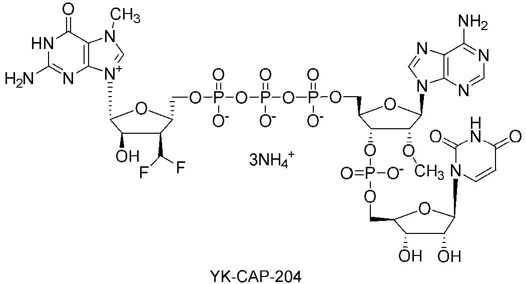 | The structure designed in the present application, synthesized in Example 1 |
| YK-CAP-205 | 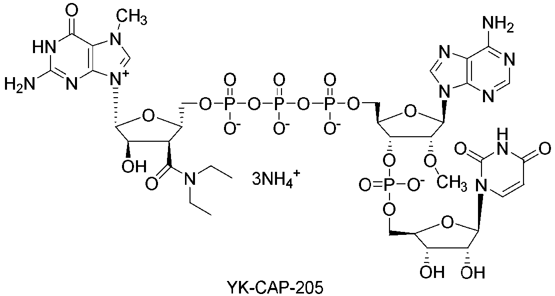 | The structure designed in the present application, synthesized in Example 1 |
| YK-CAP-206 | 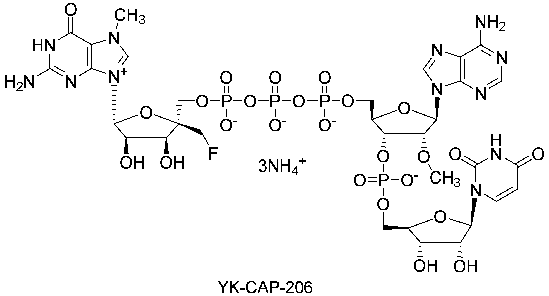 | The structure designed in the present application, synthesized in Example 1 |
| YK-CAP-207 | 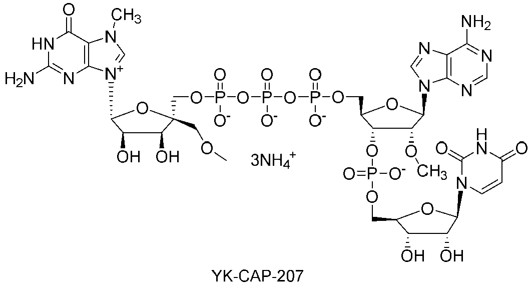 | The structure designed in the present application, synthesized in Example 1 |
| N-7114 | 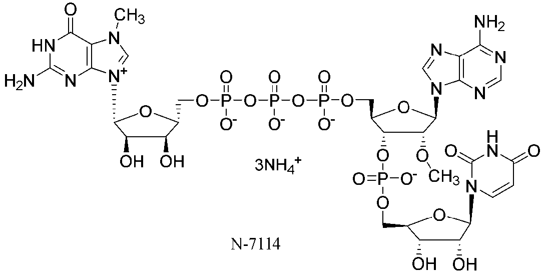 | Outsourcing (Cangzhou Weikexin Biochemical Technology Co., Ltd.) |

As shown in Table 1, the chemical structures of the compounds YK-CAP-201 to YK-CAP-207 in the present application are similar to each other, specifically as follows:
1. The compounds YK-CAP-201 to YK-CAP-207 in the present application only differ in the substituents of C3 and/or C4 on the first sugar ring.
2. The substituent of C3 on the first sugar ring of compounds YK-CAP-201 to YK-CAP-205 in the present application is different from that of N-7114: the substituent of C3 of N-7114 is hydroxyl; and the substituents of C3 of YK-CAP-201 to YK-CAP-205 are 1-methoxyethyl, 1-acetylaminoethyl, 1-fluoroethyl, difluoromethyl, and *N,N-*diethylamide, respectively.
3. The substituent of C4 on the first sugar ring of compounds YK-CAP-206 to YK-CAP-207 in the present application is different from that of N-7114: the substituent of C4 of N-7114 is hydrogen atom; and the substituents of C4 of YK-CAP-206 to YK-CAP-207 are 1-fluoromethyl and methoxymethyl, respectively.

### II. mRNA in vitro transcription yield and capping rate

### 1. Experimental method

### 1.1 Preparation of DNA Template

1) The luciferase (Luciferase protein CDS) was constructed into the pVAX1 vector (Thermo Fisher Scientific Company) via EcoRV digestion and ligation;
2) The plasmid constructed on the pVAX1 vector in step 1) was mixed with 50 µl of E. coli competent cells Stbl2 (Thermo Fisher Scientific Company) and then incubated in an ice bath for 30 minutes. The mixture was heat-shocked at 42°C for 90 seconds, immediately placed back on ice, and incubated in an ice bath for 2 minutes;
3) 400 µl of LB medium (Thermo Fisher Scientific Company) was added and incubated in a shaker at 30 °C with slow shaking for 45 to 60 minutes;
4) 50 to 100 µl of bacterial solution was spread onto LB solid medium containing kanamycin antibiotic (100 µg/ml, Yeasen Biotechnology Co., Ltd.) and incubated inverted overnight at 37°C;
5) The obtained monoclonal colony plates were sequenced to verify their accuracy. Monoclonal colonies with confirmed sequencing results were picked and cultured overnight at 30°C on a slow-shaking shaker;
6) The endotoxin-free plasmid large-scale extraction kit (MolPure^{®} Endo-free Plasmid Maxi Kit, Yeasen Biotechnology Co., Ltd., Catalog No.: 19036ES10) was used for plasmid extraction;
7) The extracted plasmid was digested into linearized plasmids using restriction endonuclease, serving as a transcription template. Refer to Steps ①- ③ below for the specific digestion procedure.

Step ①: Taking 1 mg of luciferase circular plasmid and digesting it at 37°C for 4 hours (using BspQ I enzyme, purchased from Yeasen Biotechnology Co., Ltd.) to obtain a linearized DNA transcription template (see Table 2 for the digestion reaction system);

**Table 2 Digestion reaction system**

| **Reaction components** | **Amount added** |
|---|---|
| 10 × Digestion Buffer (ml) | 2 ml |
| BspQ I enzyme | To a final concentration of 1 KU/mg |
| Luciferase circular plasmid | To a final concentration of 50 µg/ml |
| Water for injection | Supplement to a 20 ml system |

Step ②: Upon completion of the reaction, adding anhydrous ethanol and 3M sodium acetate sequentially to the enzyme digestion reaction product solution at a volume ratio of V _{enzyme digestion reaction product}: V _{anhydrous ethanol}: V _{3M sodium acetate} = 1: 3: 1, placing the mixture at -20 °C for 1 hour to precipitate, and then centrifuging at 12,000 rpm to retain the precipitate;

Step ③: Washing the precipitate obtained in step ② twice with 70% ethanol and centrifuging, drying the centrifuged material at 55°C for 10 minutes, then dissolving it in 1.7 mL of water for injection;

The concentration of linearized plasmid in the dissolution solution was 500 ng/µL, with a linearization ratio exceeding 90% and a purification recovery efficiency of 85%.

### 1.2 In vitro transcription synthesis of mRNA

### 1) Co-transcription capping reaction:

YK-CAP-201 to YK-CAP-207 and N-7114 synthesized in Example 1 were used as capping analogs, and Fluc DNA prepared in "1.1 Preparation of DNA Template" was used as the template. Nucleoside triphosphate solution (NTPs) was used as the starting material, and mRNA was synthesized by transcription using T7 RNA polymerase. The specific reaction system is shown in Table 3. The prepared reaction system was placed in a 37 °C constant temperature incubator and shaken for 3 hours.

**Table 3 Co-transcription capping reaction system**

| **Reaction components** | | **Amount added** |
|---|---|---|
| T7 transcription buffer (10×) | | T7 transcription buffer: 1/10 of the total volume of the reaction system, with a magnesium ion concentration of 40 mM. |
| NTPs | ATP | To a final concentration of 10 mM |
| | CTP | To a final concentration of 10 mM |
| | GTP | To a final concentration of 10 mM |
| | N 1-methyl-pseudouridine triphosphate or UTP | To a final concentration of 10 mM |
| Capping analogs | | To a final concentration of 10 mM |
| Linearized plasmid | | To a final concentration of 50 µg/ml |
| Murine RNase inhibitor | | To a final concentration of 1 KU/ml |
| Inorganic pyrophosphatase | | Adding 30 U per 1 mg of linearized plasmid |
| T7 RNA Polymerase | | Adding 200 KU per 1 mg of linearized plasmid |
| Water for injection | | Supplement to the target volume of the reaction system |

| | | |
|---|---|---|
| Note: N-7114 among the capping analogs was purchased from Cangzhou Weikexin Biochemical Technology Co., Ltd. Other capping analogs were synthesized according to the aforementioned content of the present application. Other reagents, excluding the capping analogs, were purchased from Yeasen Biotechnology Co., Ltd. | | |

### 2) Digestion of template DNA:

DNase I (Yeasen Biotechnology Co., Ltd.) was added to the co-transcription capping reaction system completed in step 1) above. The final concentration of DNase I was adjusted to 1 U per µg of linearized plasmid in the reaction solution. After mixing, the mixture was centrifuged and digested at 37°C for 1 hour to yield the co-transcription capping product.

### 3) Purification by lithium chloride precipitation method:

The co-transcription capping product obtained in step 2) above was purified using lithium chloride precipitation method, as detailed below:
step ① adding lithium chloride: adding lithium chloride solution (Thermo Fisher Scientific Company) to the product obtained from step 2) above to a final concentration of 2.8 M, and precipitating at low temperature for 2 hours;
step ② precipitation: centrifuging at 12,000 rpm for 15 minutes at high speed and retaining the precipitate;
Step ③ washing: washing twice with 75% ethanol, then dissolving in water for injection to obtain the mRNA solution. The purified mRNA solution was stored at -80°C.

### 1.3 Annealing between obtained mRNA and probe

Annealing in the PCR instrument: 95°C for 5 minutes; 65°C for 2 minutes; 55°C for 2 minutes; 40°C for 2 minutes; and 22°C for 2 minutes.

1.4 Magnetic bead pretreatment and probe binding: 100 µl of magnetic beads were placed on a magnetic rack for pretreatment. 120 µl of the sample was added and incubated with magnetic bead solution at room temperature for 30 minutes, while slowly mixing.

### 1.5 Cleavage of mRNA to obtain the 5' single-stranded sequence of mRNA bound to the probe

20 µl of RnaseH (5 U/µl) was added and incubated at 37 °C for 3 hours, mixing evenly every half hour. After incubation, the magnetic beads were washed. Following washing, 100 µl of 75% methanol heated to 80°C was added to the washed magnetic beads. The resulting mixture was heated to 80°C on a heating plate and maintained for 3 minutes, then placed on a magnetic rack to aspirate the supernatant, which was dried using an evaporative centrifuge at room temperature for 45 minutes to a volume of 10 µl. The sample was then resuspended in 50 µl of 100 µM EDTA/1% MeOH and was ready for LC-MS analysis to determine RNA capping status in the transcription reaction. Since capped and uncapped bases exhibit distinct molecular weights, the capping rate of mRNA transcription initiated by different capping analogs can be determined by using these mass differences.

### (6) Evaluation of mRNA integrity using Agilent 5200 Fragment Analyzer capillary electrophoresis system.

1) Preparation of sample: Based on the sample concentrations determined earlier using an ultra-micro spectrophotometer, each sample was diluted to 20 ng/µl to 100 ng/µl at different dilution ratios with RNase-free water. The samples and pre-aliquoted Ladder were mixed evenly and placed in a metal bath device (Hangzhou Yooning, MiniH-100D). Denaturation was performed at 70°C for 2 minutes, after which the samples were immediately transferred to ice.
2) Preparation of capillary reagents:

**Table 4 Preparation of capillary reagents**

| Preparation of reagents | |
|---|---|
| Preparation of RNA Separation Gel | 30 ml of Separation Gel and 3.0 µl of Intercalationg Dye were pipetted into a 50 ml centrifuge tube. The tube was inverted to mix evenly, avoiding the formation of bubbles. |
| Preparation of 1x Capillary Conditioning Solution | 6 ml of 5x Capilary Conditioning Solution and 24 ml of RNase-free water were pipetted into a 50 ml centrifuge tube. The tube was inverted to mix evenly, avoiding the formation of bubbles. |
| Preparation of 1x930 dsDNA Inlet Buffer | 3 ml of 5x 930 dsDNA Inlet Buffer and 12 ml of RNase-free water were pipetted into a 50 ml centrifuge tube. The tube was inverted to mix evenly, avoiding the formation of bubbles. The prepared solution was added to row A of |
| | the deep well plate at 1 ml per well. The deep well plate was then placed in the "B" drawer of the instrument. |
| 0.25x× TE Rinse Buffer | 0.25x TE Rinse Buffer was added to row A of the sample plate, with 200 µl per well, and the sample plate was placed in the "M" drawer of the instrument. |
| Preparation of sample plate | 2 µl of the solution to be tested or Ladder and 22 µl of Diluent Marker were pipetted into the sample plate to obtain a mixture, the mixture was mixed evenly by pipetting up and down 10 to 15 times. |

3) Setting the sample operation program and perform sample injection and measurement: Setting the injection voltage for the sample to 5 kV, operation time to 4 s, separation voltage to 8 kV, and operation time to 45 min.
4) Data analysis: Agilent Fragment Analyzer came with ProSize analysis software, enabling users to perform batch calculations of the percentage of RNA fragments meeting the requirements or the percentage of degraded RNA through Smear analysis.

### 2. Experimental results

The measurement results of mRNA *in vitro* transcription yield, integrity, and capping rate showed that the capping rate was basically consistent and at a very high level. Compared with the capping analogs for self-replicating mRNA in the prior art, the capping analog for self-replicating mRNA in the present disclosure significantly enhances both mRNA *in vitro* transcription yield and integrity, while achieving a very high capping rates. The specific mRNA *in vitro* transcription yield and capping rate are shown in Table 5.

**Table 5 Measurement results of co-transcription capping reaction system**

| Name | Unit template yield (µg) | Improvement relative to N-7114 (%) | Integrity (%) | Improvement relative to N-7114 (%) | Capping rate (%) | Sequence length (bp) |
|---|---|---|---|---|---|---|
| YK-CAP-201 | 176.3 | 0.6 | 69.8 | 16.1 | 98.6 | 7,439 |
| YK-CAP-202 | 111.1 | -36.6 | 50.3 | -16.3 | 93.0 | 7,546 |
| YK-CAP-203 | 182.0 | 3.8 | 64.3 | 7.0 | 99.6 | 7,406 |
| YK-CAP-204 | 204.8 | 16.8 | 70.9 | 18.0 | 96.6 | 7,520 |
| YK-CAP-205 | 179.8 | 2.6 | 65.8 | 9.5 | 97.8 | 7,566 |
| YK-CAP-206 | 207.9 | 18.6 | 71.0 | 18.1 | 98.1 | 7,500 |
| YK-CAP-207 | 83.6 | -52.3 | 34.0 | -43.4 | 99.0 | 7,298 |
| N-7114 | 175.3 | 0 | 60.1 | 0 | 98.4 | 7,457 |

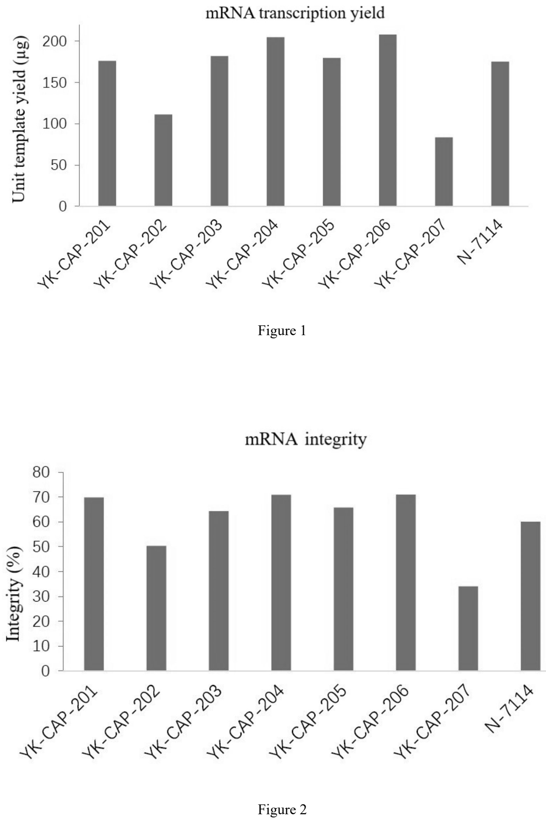

As shown in Table 5, the capping analogs for self-replicating mRNA in the present application all efficiently transcribe mRNA (Figure 1). Compared with the capping analogs for self-replicating mRNA in the prior art, the *in vitro* transcription yield and integrity of mRNA are significantly improved.

The mRNA *in vitro* transcription yield and integrity of N-7114 (Figure 2) were 175.3 µg and 60.1%, respectively. The mRNA *in vitro* transcription yield and integrity of YK-CAP-206 in the present application were increased by 18.6% and 18.1%, respectively, compared to N-7114.

The *in vitro* transcription yield and integrity of mRNA demonstrate that, compared to N-7114 in the prior art, the *in vitro* transcription yield and integrity of mRNA produced by the capping analogs for self-replicating mRNA in the present application are significantly enhanced. This demonstrates that the capping analog in the present application exhibits superior anti-reverse transcription activity when used for self-replicating mRNA and is capable of enhancing the binding capacity between the cap structure and the capping enzyme, thereby increasing the capping rate of transcribed mRNA.

### Example 3: Preparation and characterization of lipid nanoparticles

### 1. Experimental method

Cationic lipid YK-009 (Beijing Youcare Kechuang Pharmaceutical Technology Co., Ltd.), DSPC (AVT (Shanghai) Pharmaceutical Technology Co., Ltd.), cholesterol (AVT (Shanghai) Pharmaceutical Technology Co., Ltd.), and DMG-PEG 2000 were dissolved in ethanol in a molar ratio of 49: 10: 39.5: 1.5, and mRNA was diluted in 50 mM citrate buffer, pH = 4. Using a microfluidic device, the ethanol lipid solution was mixed with the aqueous FLuc mRNA solution prepared with different cap structures at a flow rate of 10 ml/min in a 1: 3 volume ratio. LNP was prepared with a total lipid-to-mRNA mass ratio of approximately 15: 1. After diluting the obtained liposomes with PBS to 10 times the volume, ethanol was filtered out using a 300 KDa ultrafiltration tube. The solution was then diluted to a specific volume with PBS. Finally, the lipid nanoparticles were filtered through a 0.2 µm sterile filter to obtain the LNP formulation of YK-009/DSPC/cholesterol/DMG-PEG2000 (mole ratio 49: 10: 39.5: 1.5) encapsulating self-replicating FLuc-mRNA.

The structural formula of YK-009 is as follows:

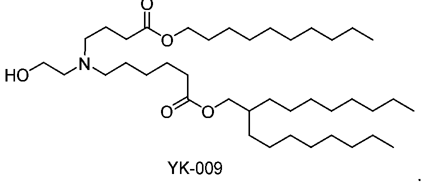

Using dynamic light scattering, particle size and polydispersity index (PDI) were determined with a Malvern laser diffraction particle size analyzer. 25 µl of the liposome solution was diluted with physiological saline to 125 µl and added to the sample cell. Each sample was measured in triplicate. Measurement conditions: 90° scattering angle, 25 °C. According to the manufacturer's instructions, the encapsulation efficiency of lipid nanoparticles was determined using Quant-it Ribogreen RNA Quantification Assay Kit (Thermo Fisher Scientific, UK).

### 2. Experimental results

The specific characterization data for the lipid nanoparticles are shown in Table 6.

**Table 6 Characterization of lipid nanoparticles**

| Name | Particle size (nm) | PDI | Encapsulation rate (%) |
|---|---|---|---|
| YK-CAP-201 | 77.15 | 0.075 | 95.6 |
| YK-CAP-202 | 77.85 | 0.076 | 94.9 |
| YK-CAP-203 | 74.73 | 0.064 | 95.0 |
| YK-CAP-204 | 77.30 | 0.058 | 93.8 |
| YK-CAP-205 | 73.61 | 0.073 | 93.3 |
| YK-CAP-206 | 82.98 | 0.052 | 93.9 |
| YK-CAP-207 | 71.10 | 0.031 | 93.9 |
| N-7114 | 77.09 | 0.095 | 93.1 |

As shown in Table 6, self-replicating FLuc-mRNA transcribed from the capping analogs YK-CAP-201 to YK-CAP-207 in the present application and the capping analog N-7114 disclosed in the prior art can all be used to prepare well-formed lipid nanoparticles. All lipid nanoparticles have a particle size between 70 nm and 85 nm, a PDI value between 0.031 and 0.096, and an encapsulation rate of over 90%.

### Example 4: Translation efficiency of luciferase mRNA containing different cap structures

### 1. Experimental method

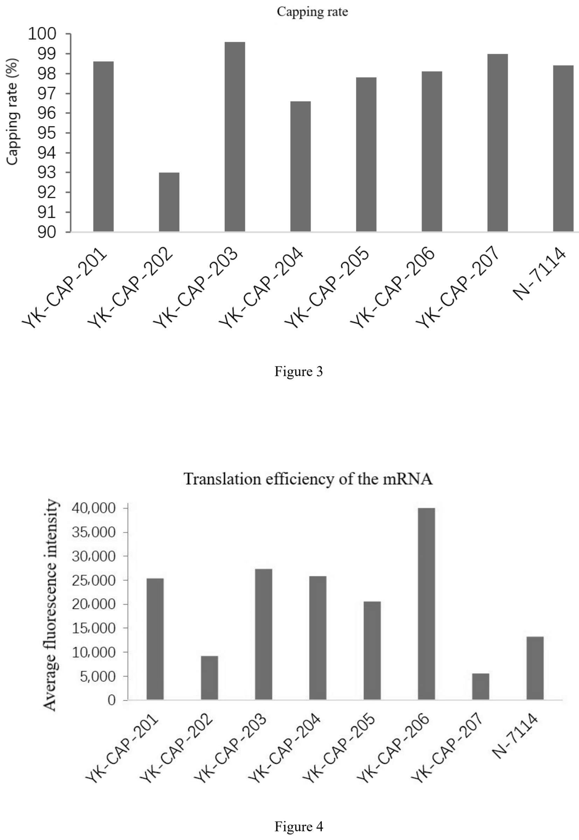

(1) HEK293T cells was cultured in DMEM medium supplemented with 10% FBS and penicillin/streptomycin at 37 °C under 5% CO₂ conditions.
(2) The cells in the culture dish were digested and counted, then plated in a 96 well plate at 10,000 cells per well and cultured overnight until the cells adhered to the wall.
(3) When the cell density is approximately 80%, transfection was performed by adding 50 ng of mRNA sample and Lipofectamine Messenger MAX Transfection Reagent (Invitrogen) reagent to each well. The transfection steps were performed according to the instructions.
(4) After transfection, the cells were further cultured at 37 °C with 5% CO₂ for 24 hours. The growth medium was removed from the cells to be tested, and the cells were washed with PBS. After centrifugation to remove PBS, 50 µl of 1x lysis buffer was added, the cells and all the liquid were transferred to a micro-centrifuge tube, and then centrifuged.
(5) 100 µl of Dual-Lumi^{™} II Firefly Luciferase Assay Reagent, equilibrated to room temperature, was added to 20 µl of sample and mixed appropriately.
(6) The mixture was incubated at room temperature (approximately 25 °C) for 5 minutes to allow the luminescence signal to stabilize. A multifunctional microplate reader equipped with chemiluminescence detection function was employed to perform chemiluminescence assays, and the data were recorded (see Table 7 and Figure 4).

### 2. Experimental results

The relative fluorescence readings for capping mRNA are shown in Table 7. The relative fluorescence intensity is directly proportional to the translation efficiency of the mRNA.

**Table 7 Average fluorescence readings of capping mRNA**

| Name | Average fluorescence intensity | Multiple relative to N-7114 |
|---|---|---|
| YK-CAP-201 | 25,377 | 1.9 |
| YK-CAP-202 | 9,182 | 0.7 |
| YK-CAP-203 | 27,330 | 2.1 |
| YK-CAP-204 | 25,834 | 2.0 |
| YK-CAP-205 | 20,553 | 1.6 |
| YK-CAP-206 | 40,045 | 3.0 |
| YK-CAP-207 | 5,568 | 0.4 |
| N-7114 | 13,241 | - |

It can be seen that the capping analogs for self-replicating mRNA in the present application have significantly improved mRNA translation efficiency compared to the capping analogs for self-replicating mRNA in the prior art.

The average fluorescence intensity of N-7114 (corresponding to the translation efficiency of mRNA) is 13,241. The average fluorescence intensity of YK-CAP-206 is 3.0 times that of N-7114, and the average fluorescence intensity of YK-CAP-203 is 2.1 times that of N-7114.

The substituent of C3 on the first sugar ring of YK-CAP-201 to YK-CAP-205 in the present application is different from that of N-7114: the substituent of C3 of N-7114 is hydroxyl; and the substituents of C3 of YK-CAP-201 to YK-CAP-205 are 1-methoxyethyl, 1-acetylaminoethyl, 1-fluoroethyl, difluoromethyl, and *N,N*-diethylamide, respectively. However, the translation efficiencies of the corresponding mRNAs for YK-CAP-201, YK-CAP-203, YK-CAP-204, and YK-CAP-205 were 1.9-fold, 2.1-fold, 2.0-fold, and 1.6-fold that of N-7114, respectively.

From the translation efficiency of different capping luciferase mRNA, it can be seen that the capping analogs for self-replicating mRNA in the present application have significantly improved mRNA translation efficiency compared to the capping analogs N-7114 for self-replicating mRNA in the prior art. This indicates that modifying ribose with the capping analog in the present application is capable of facilitating binding to the cap-binding protein EIF4E, thereby enhancing the translation efficiency of target mRNA.

### Example 5: Cell viability assay

### 1. Experimental method

The LNP formulation containing 1 µg of self-replicating FLuc-mRNA, prepared according to Example 3, was added to the cell culture medium in a 96-well plate. After 24 hours of incubation, 10 µl of CCK-8 solution was added to each well. The culture plate was incubated in an incubator for 1 hour, followed by measurement of the absorbance at 450 nm using a microplate reader. The results are shown in Table 8.

Cell viability can indicate the toxicity of transcribed self-replicating FLuc-mRNA to cells. A higher cell viability signifies lower toxicity to the cells.

### 2. Experimental results

**Table 8 Cell viability**

| Name | Cell viability (%) |
|---|---|
| YK-CAP-201 | 94 |
| YK-CAP-202 | 99 |
| YK-CAP-203 | 100 |
| YK-CAP-204 | 98 |
| YK-CAP-205 | 97 |
| YK-CAP-206 | 94 |
| YK-CAP-207 | 98 |
| N-7114 | 96 |

It can be seen that both the self-replicating FLuc-mRNA transcribed using the capping analogs YK-CAP-201 to YK-CAP-207 of the present application and the capping analog N-7114 of the prior art have relatively low cytotoxicity, with cell viability greater than 90%.

### Example 6: Animal experiment

### 1. Experimental method

Female BALB/c mice aged 4 to 6 weeks and weighing 17 to 19 g were intramuscularly injected with an LNP formulations containing 5 µg of the self-replicating FLuc-mRNA transcribed with the capping analogs. Fluorescent imaging substrates were then injected intraperitoneally into the mice at 24 h, 48 h, 72 h, 120 h, and 192 h post-administration. After 5 minutes of free activity, the total radiant intensity of the protein expressed *in vivo* (in mice) from the mRNA carried by the LNP was detected using an IVIS Spectrum small animal *in vivo* imaging system, which reflects the level of protein expression.

### 2. Experimental results

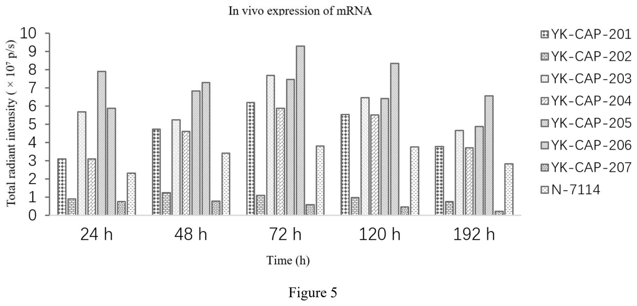

Test results are shown in Table 9 and Figure 5. In the live imaging experiment of mice, the multiples of the total radiant intensity of each group of mice relative to N-7114 are shown in Table 10 (where the total radiation intensity is the stated value × 10⁷ p/s).

**Table 9 Experimental data of live imaging of mice**

| Name | Total radiant intensity ( × 10⁷ p/s) | | | | |
|---|---|---|---|---|---|
| | 24 h | 48 h | 72 h | 120 h | 192 h |
| YK-CAP-201 | 3.09 | 4.73 | 6.19 | 5.55 | 3.79 |
| YK-CAP-202 | 0.90 | 1.25 | 1.09 | 0.97 | 0.76 |
| YK-CAP-203 | 5.68 | 5.26 | 7.68 | 6.47 | 4.67 |
| YK-CAP-204 | 3.11 | 4.61 | 5.88 | 5.51 | 3.71 |
| YK-CAP-205 | 7.90 | 6.83 | 7.48 | 6.43 | 4.88 |
| YK-CAP-206 | 5.88 | 7.30 | 9.31 | 8.36 | 6.56 |
| YK-CAP-207 | 0.76 | 0.78 | 0.58 | 0.46 | 0.23 |
| N-7114 | 2.33 | 3.42 | 3.82 | 3.77 | 2.83 |

**Table 10 Multiples of total radiant intensity relative to N-7114**

| Name | 24 h | 48 h | 72 h | 120 h | 192 h |
|---|---|---|---|---|---|
| YK-CAP-201 | 1.3 | 1.4 | 1.6 | 1.5 | 1.3 |
| YK-CAP-202 | 0.4 | 0.4 | 0.3 | 0.3 | 0.3 |
| YK-CAP-203 | 2.4 | 1.5 | 2.0 | 1.7 | 1.7 |
| YK-CAP-204 | 1.3 | 1.3 | 1.5 | 1.5 | 1.3 |
| YK-CAP-205 | 3.4 | 2.0 | 2.0 | 1.7 | 1.7 |
| YK-CAP-206 | 2.5 | 2.1 | 2.4 | 2.2 | 2.3 |
| YK-CAP-207 | 0.3 | 0.2 | 0.2 | 0.1 | 0.1 |
| N-7114 | 1.0 | 1.0 | 1.0 | 1.0 | 1.0 |

Compared to capping analogs in the prior art, the total radiant intensity and duration of the protein expressed in mice from mRNA transcribed using the capping analog of the present application are significantly enhanced. For example, the corresponding total radiant intensity of YK-CAP-206 is 2.5 times, 2.1 times, 2.4 times, 2.2 times, and 2.3 times that of N-7114 at 24 h, 48 h, 72 h, 120 h, and 192 h, respectively. The total radiant intensity of YK-CAP-205 at corresponding times was 3.4 times, 2.0 times, 2.0 times, 1.7 times, and 1.7 times that of N-7114, respectively.

The substituent of C3 on the first sugar ring of compounds YK-CAP-201 to YK-CAP-205 in the present application is different from that of N-7114, that is, the substituent of C3 of N-7114 is hydroxyl; the substituents of C3 of YK-CAP-201 to YK-CAP-205 are 1-methoxyethyl, 1-acetylaminoethyl, 1-fluoroethyl, difluoromethyl, and *N, N*-diethylamide, respectively. However, the total radiant intensity of proteins expressed in mice at 24 hours for YK-CAP-201, YK-CAP-203, YK-CAP-204, and YK-CAP-205 was 1.3 times, 2.4 times, 1.3 times, and 3.4 times that of N-7114, respectively, and 3.4 times, 6.3 times, 3.5 times, and 8.8 times that of YK-CAP-202, respectively.

From animal experiments, it can be seen that compared to the capping analog N-7114 in the prior art, the mRNA transcribed using the capping analog of the present application exhibited significantly increased protein expression level and duration in mice. *In vivo* experiments further demonstrated that the mRNA transcribed by YK-CAP-201 and YK-CAP-203 to YK-CAP-206 of the present application can be effectively delivered into the body via LNP delivery vectors and expressed efficiently and sustainably.

In summary, the capping analogs of the present application, YK-CAP-201 and YK-CAP-203 to YK-CAP-206, when used for self-replicating mRNA, demonstrate significant improvements in terms of mRNA *in vitro* transcription yield, integrity, capping rate, mRNA translation efficiency, as well as *in vivo* protein expression level and duration in animals compared to the capping analog N-7114 for self-replicating mRNA in the prior art. This indicates that the capping analogs provided in the present disclosure can significantly enhance the affinity for binding to capping enzymes, thereby providing a cap structure for *in vitro* transcription of mRNA that is highly effective for self-replicating mRNA.
1. Compared to the capping analogs for self-replicating mRNA in the prior art, the capping analog for self-replicating mRNA in the present application significantly enhances both mRNA *in vitro* transcription yield and integrity. For example, the mRNA *in vitro* transcription yield and integrity of N-7114 were 175.3 µg and 60.1%, respectively. The mRNA *in vitro* transcription yield and integrity of YK-CAP-206 in the present application were increased by 18.6% and 18.1%, respectively, compared to N-7114.
2. Compared to the capping analogs for self-replicating mRNA in the prior art, the capping analog for self-replicating mRNA in the present application significantly enhances mRNA translation efficiency. For example, the translation efficiency of YK-CAP-206 in the present application reaches 3.0 times that of N-7114.

The above examples are provided merely to illustrate the technical solutions disclosed herein and are not intended to limit the scope of the present disclosure. Although the present disclosure has been described in detail with reference to the foregoing examples, those skilled in the art should understand that it is still possible to amend the technical solutions recorded in the foregoing examples, or to make equivalent substitutions for some of or all of the technical features therein. Any modification, equivalent replacement or improvement made within the spirit and principle of the present disclosure should be included in the protection scope of the claims in the present disclosure.

## Claims

1. A compound represented by formula (I), a stereoisomer thereof or a pharmaceutically acceptable salt thereof; 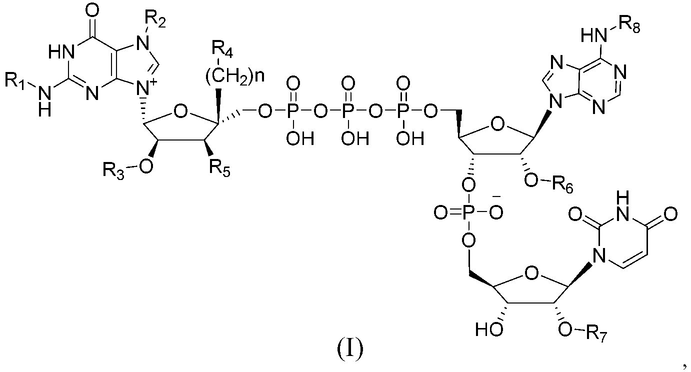
where n is 0, 1, 2 or 3;
R₅ is -OR₅ₐ, -SR_{5b}, -NR_{5c}R_{5d}, -C(O)R_{5c}, 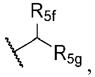 -NHC(O)-R₅ₕ, -N(CH₃)C(O)-R₅ᵢ, -C(O)NR₅ⱼR₅ₖ, -C(O)O-R₅ₗ, -OC(O)-R₅ₘ or -S(O)₂-R₅ₙ;
R₄ₐ, R₅ₐ, R_{5b}, R_{5c}, R_{5d}, R_{5c}, R₅ₕ, R₅ᵢ, R₅ⱼ, R₅ₖ, R₅ₗ, R₅ₘ and R₅ₙ are independently -H, C₁-C₃ alkyl or halogenated C₁-C₃ alkyl;
R_{5f} and R_{5g} are independently -H, halogen, C₁-C₃ alkyl, -OC₁-C₃ alkyl, halogenated C₁-C₃ alkyl or -NR₅₋₁C(O)R₅₋₂;
R₅₋₁ and R₅₋₂ are independently -H or C₁-C₃ alkyl;
R₁, R₂, R₃, R₆ and R₇ are each independently -H, C₁-C₆ alkyl, C₂₋C₆ alkenyl, C₂-C₆ alkynyl, C₃-C₆ cycloalkyl or benzyl;
R₄ is -OR₄ₐ, -F or -H; and
R₈ is C₁-C₃ alkyl or -H;
preferably, the compound represented by formula (I) satisfies any one or a combination of at least two of the following conditions (1) to (4):
(1) R₄ is -H, -OC₁-C₃ alkyl or halogen;
(2) R₅ is -OH, 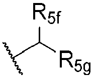 or -C(O)NR₅ⱼR₅ₖ; R_{5f} and R_{5g} are independently halogen C₁-c₃ alkyl -OC₁-C₃ alkyl or -NR₅₋₁C(O)R₅₋₂; R₅ⱼ and R₅ₖ are independently C₁-C₃ alkyl; R₅₋₁ is H; and R₅₋₂ is C₁-C₃ alkyl;
(3) R₂ is C₁-C₃ alkyl; and
(4) R₆ is C₁-C₃ alkyl, or the compound represented by formula (I) satisfies any one or a combination of at least two of the following conditions (1) to (9):
(1) R₄ is -H, -F or -OCH₃;
(2) R₅ is -OH, 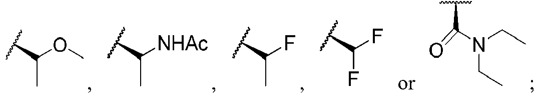
(3) R₁ is -H;
(4) R₂ is -CH₃;
(5) R₃ is -H;
(6) R₆ is -CH₃;
(7) R₇ is -H;
(8) R₈ is -H; and
(9) n is 0 or 1;
more preferably, n is 0 or 1; R₄ is -H or halogen;
R₅ is -OH, 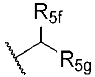 or -C(O)NR₅ⱼR₅ₖ; R_{5f} and R_{5g} are independently selected from halogen, C₁-C₃ alkyl or -OC₁-C₃ alkyl; R₅ⱼ and R₅ₖ are independently C₁-C₃ alkyl;
R₁ is -H; R₂ is C₁-C₃ alkyl; R₃ is -H;
R₆ is C₁-C₃ alkyl; R₇ is -H; and
R₈ is -H.

2. The compound represented by formula (I), a stereoisomer thereof or a pharmaceutically acceptable salt thereof according to claim 1, wherein the pharmaceutically acceptable salt of the compound represented by formula (I) has any of the following structures: 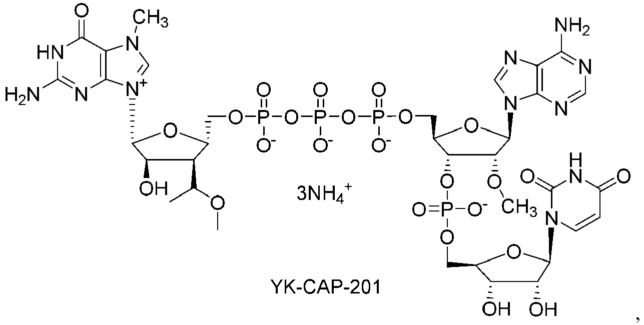 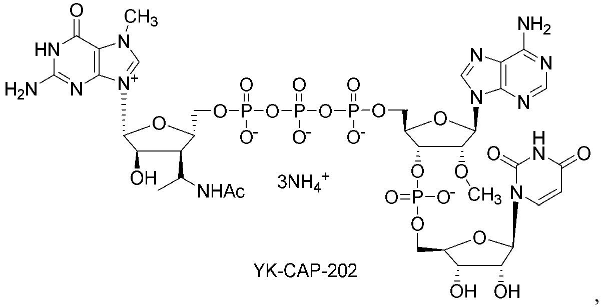 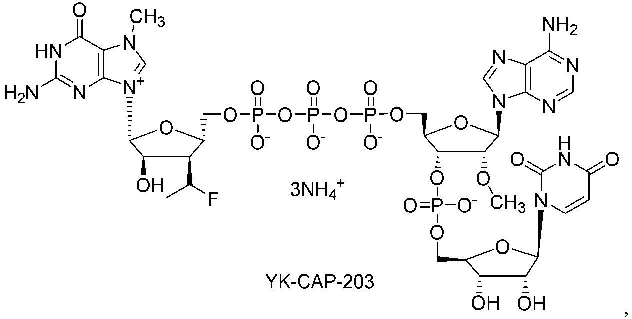 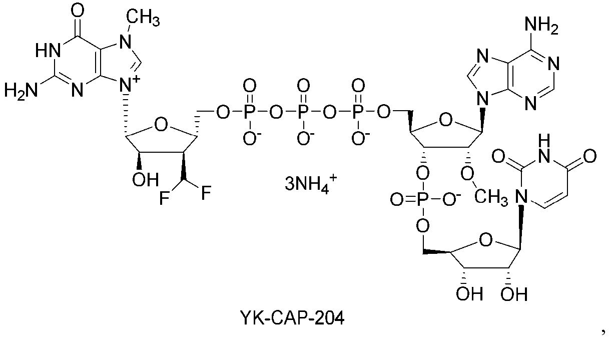 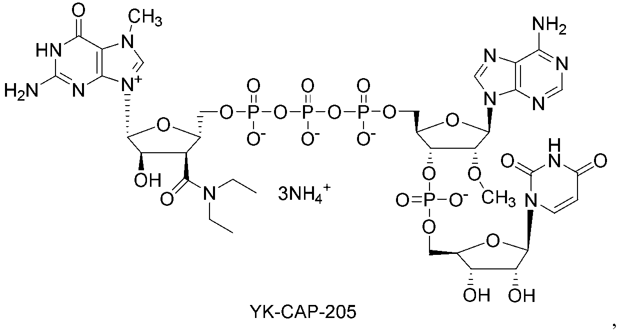 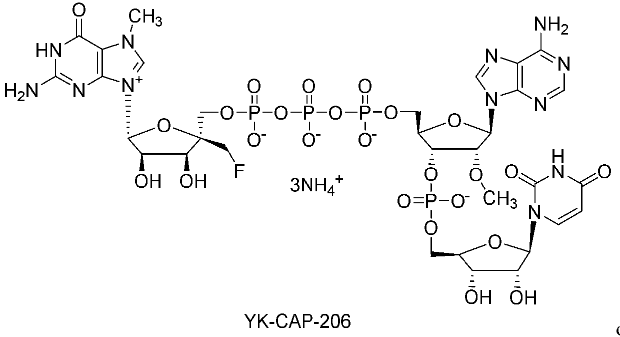 or

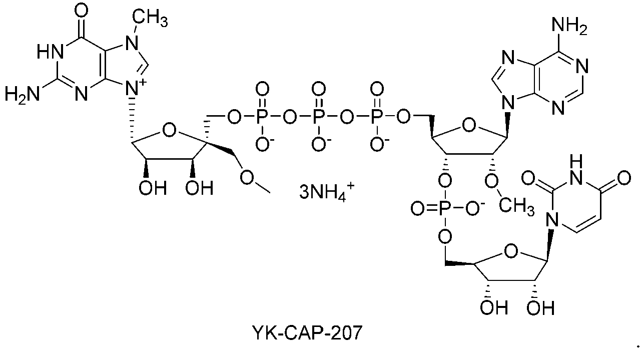

3. Use of the compound represented by formula (I), a stereoisomer thereof or a pharmaceutically acceptable salt thereof according to claim 1 or2, in the preparation of an *in vitro* co-transcription mRNA capping reagent.

4. An RNA molecule comprising the compound represented by formula (I), a stereoisomer thereof or a pharmaceutically acceptable salt thereof according to claim 1 as a cap structure or cap structure fragment.

5. A pharmaceutical composition comprising the RNA molecule according to claim 4.

6. The pharmaceutical composition according to claim 5, wherein the pharmaceutical composition further comprises at least one delivery agent; preferably, the at least one delivery agent is selected from the group consisting of cationic lipids, neutral lipids, structural lipids and polymer conjugated lipids, or a combination of at least two thereof.

7. The pharmaceutical composition according to claim 6, wherein the pharmaceutical composition satisfies any one or a combination of at least two of the following conditions (1) to (4):
(1) the cationic lipid is any one or a combination of at least two of the following substances:
(i) a compound represented by formula (II), a stereoisomer thereof, a N-oxide thereof, a solvate thereof or a pharmaceutically acceptable salt thereof, wherein G₁ is C₁₋₆ alkylene; G₂ is C₂₋₈ alkylene; G₃ is C₁₋₃ alkylene; L₁ is C₆₋₁₅ linear alkyl; and L₂ is C₁₂₋₂₅ branched alkyl; 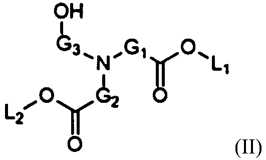
(ii) a compound represented by formula (III), a stereoisomer thereof, a N-oxide thereof, a solvate thereof or a pharmaceutically acceptable salt thereof, wherein G₁ is C₂₋₈ alkylene; G₂ is C₂₋₈ alkylene; L₁ is -C(O)O- or -OC(O)-; L₂ is -C(O)O- or -OC(O)-; R₁ is C₆₋₂₅ linear or branched alkyl; R₂ is C₆₋₂₅ linear or branched alkyl; G₃ is HO(CH₂)₂- or HO(CH₂)₃-; G₄ is HO(CH₂)₂- or HO(CH₂)₃-; and L is (CH₂)₂-, -(CH₂)₃- or -(CH₂)₄-; 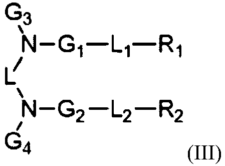
(iii) a compound represented by formula (IV), a stereoisomer thereof, a N-oxide thereof, a solvate thereof or a pharmaceutically acceptable salt thereof, wherein G₁ is C₁₋₆ alkylene; G₂ is C₂₋₈ alkylene; R₁ is C₆₋₂₀ linear or branched alkyl; R₂ is C₁₂₋₂₅ branched alkyl; and G₃ is HO(CH₂)₂N(CH₃)(CH₂)₂-, HO(CH₂)₂N(CH₂CH₃)(CH₂)₂-, (HO(CH₂)₂)₂N(CH₂)₂-, CH₃0(CH₂)₂N(CH₃)(CH₂)₂-, (CH₃)₂N(CH₂)₃SC(O)O(CH₂)₂-, (CH₃)₂N(CH₂)₃SC(O)-, CH₃NH(CH₂)₂N(CH₃)(CH₂)₂- or CH₃CH₂NH(CH₂)₂-; 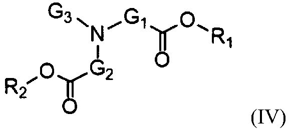
(iv) a compound represented by formula (V), a stereoisomer thereof, a N-oxide thereof, a solvate thereof or a pharmaceutically acceptable salt thereof, wherein G₁ is C₁₋₈ alkylene; G₂ is C₂₋₈ alkylene; R₁ is C₆₋₂₅ linear or branched alkyl; R₂ is C₁₂₋₂₅ linear or branched alkyl; and G₃ is HO(CH₂)₂N(R₃)CH₂CH(OH)CH₂-, where R₃ is -CH₃, -CH₂CH₃ or -CH₂CH₂OH; 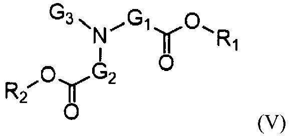
(v) a compound represented by formula (VI), a stereoisomer thereof, a N-oxide thereof, a solvate thereof or a pharmaceutically acceptable salt thereof, wherein G¹ and G² are each independently C₆-C₁₀ alkylene; G³ is C₁-C₁₂ alkylene; R¹ and R² are each independently C₆-C₂₄ alkyl or C₆-C₂₄ alkenyl; R³ is OR⁵, N(R⁵)₂, -C( = O)OR⁴, -OC( = O)R⁴ or -NR⁵C( = O)R⁴; R⁴ is C₁-C₁₂ hydrocarbyl; and R⁵ is H or C₁-C₆ hydrocarbyl; 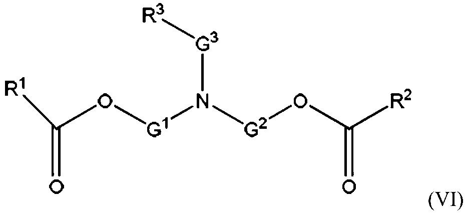
(vi) a compound represented by formula (VII), a stereoisomer thereof, a N-oxide thereof, a solvate thereof or a pharmaceutically acceptable salt thereof, wherein R₄ is -(CH₂)ₙQ; Q is -OR, -OH, -O(CH₂)ₙN(R)₂, -OC(O)R, -CX₃, -CN, -N(R)C(O)R, -N(H)C(O)R, -N(R)S(O)₂R, -N(H)S(O)₂R, -N(R)C(O)N(R)₂, -N(H)C(O)N(R)₂, -N(H)C(O)N(H)(R), -N(R)C(S)N(R)₂, -N(H)C(S)N(R)₂, -N(H)C(S)N(H)(R), -N(R)S(O)₂R or heterocyclyl; n is 1, 2 or 3; R is independently C₁₋₈ alkyl; and X is independently H or C₁₋₈ alkyl; 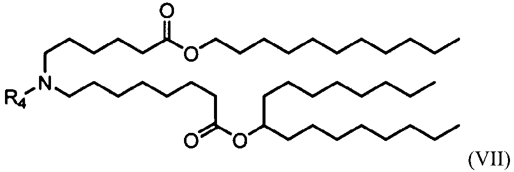
(vii) a compound represented by formula (VIII), a stereoisomer thereof, a N-oxide thereof, a solvate thereof or a pharmaceutically acceptable salt thereof, 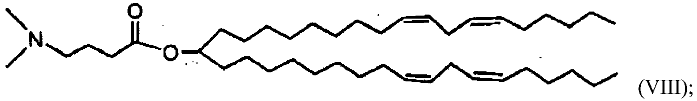
(2) the neutral lipid is selected from the group consisting of phosphatidylcholine, phosphatidylethanolamine, sphingomyelin, ceramide and sterol, or a combination of at least two thereof;
(3) the structural lipid is selected from the group consisting of cholesterol, nonsterol, sitosterol, ergosterol, campesterol, stigmasterol, brassicasterol, tomatine, ursolic acid, α-tocopherol and corticosteroid, or a combination of at least two thereof; and
(4) the polymer conjugated lipid is selected from the group consisting of 1,2-distearoyl-sn-glycero-3-phosphoethanolamine-N-[methoxy(polyethylene glycol)-2000 1,2-dimyristoyl-sn-glycero-3-methoxypolyethylene glycol 2000 and methoxypoly(ethylene glycol) ditetradecylacetamide, or a combination of at least two thereof; preferably, the pharmaceutical composition satisfies any one or a combination of at least two of the following conditions (1) to (4):
(1) the cationic lipid is selected from the group consisting of YK-009, YK-201, YK-407, YK-305, ALC-0315, SM-102 and DLIN-MC3-DMA, or a combination of at least two thereof: 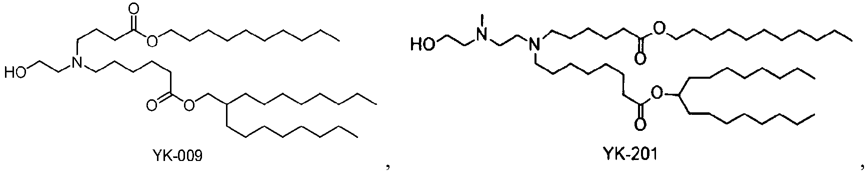 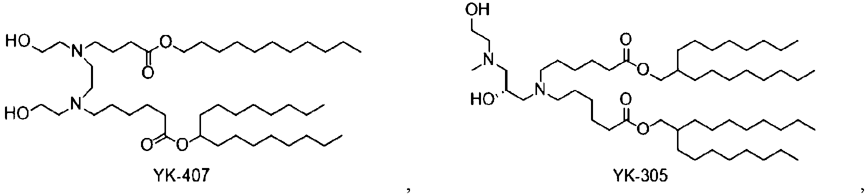 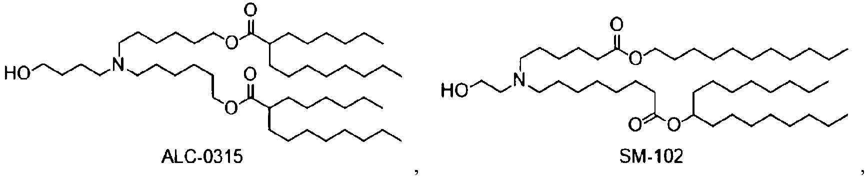 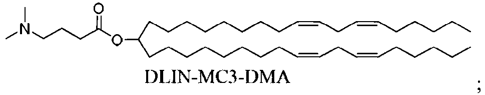
(2) the neutral lipid is selected from the group consisting of 1,2-dilinoleoyl-sn-glycero-3-phosphocholine, 1,2-dimyristoyl-sn-glycero-phosphorylcholine, 1,2-dioleoyl-sn-glycero-3-phosphocholine, 1,2-dipalmitoyl-sn-glycero-3-phosphocholine, 1,2-distearoyl-sn-glycero-3-phosphocholine, 1,2-diundecanoyl-sn-glycero-phosphocholine, 1-palmitoyl-2-oleoyl-sn-glycero-3-phosphocholine, 1,2-di-O-octadecenyl-sn-glycero-3-phosphocholine, 1-oleoyl-2-cholesterylhemisuccinoyl-sn-glycerol-3-phosphocholine, 1-hexadecyl-sn-glycero-3-phosphocholine, 1,2-dilinolenoyl-sn-glycero-3-phosphocholine, 1,2-diarachidonoyl-sn-glycero-3-phosphocholine, 1,2-bis(docosahexaenoyl)-sn-glycero-3-phosphocholine, 1,2-dioleoyl-sn-glycerol-3-phosphoethanolamine, 1,2-diphytanoyl-sn-glycero-3-phosphoethanolamine, 1,2-distearoyl-sn-glycero-3-phosphoethanolamine, 1,2-dilinoleoyl-sn-glycero-3-phosphoethanolamine, 1,2-dilinolenoyl-sn-glycero-3-phosphoethanolamine, 1,2-diarachidonoyl-sn-glycero-3-phosphoethanolamine, 1,2-bis(docosahexaenoyl)-sn-glycero-3-phosphoethanolamine, 1,2-dioleoyl-sn-glycero-3-phosphate-rac-(1-glycerol) sodium salt, dipalmitoyl phosphatidylglycerol, palmitoyl oleoyl phosphatidylethanolamine, distearoyl-phosphatidyl-ethanolamine, dipalmitoyl phosphatidyl ethanolamine, dimyristoyl phosphoethanolamine, 1-stearoyl-2-oleoyl-stearoyl-ethanolamine, 1-stearoyl-2-oleoyl-phosphatidylcholine, sphingomyelin, phosphatidylcholine, phosphatidylethanolamine, phosphatidylserine, phosphatidylinositol, phosphatidic acid, palmitoyl oleoyl phosphatidylcholine, lysophosphatidylcholine and lysophosphatidylethanolamine, or a combination of at least two thereof;
(3) the structural lipid is cholesterol; and
(4) the polymer conjugated lipid is 1,2-dimyristoyl-sn-glycero-3-methoxypolyethylene glycol 2000;
more preferably, the cationic lipid is the YK-009; and/or the neutral lipid is 1,2-dioleoyl-sn-glycero-3-phosphoethanolamine and/or 1,2-distearoyl-sn-glycero-3-phosphocholine.

8. The pharmaceutical composition according to claim 6, wherein the at least one delivery agent comprises YK-009, 1,2-distearoyl-sn-glycero-3-phosphocholine, cholesterol and 1,2-dimyristoyl-sn-glycero-3-methoxypolyethylene glycol 2000; and the YK-009 is 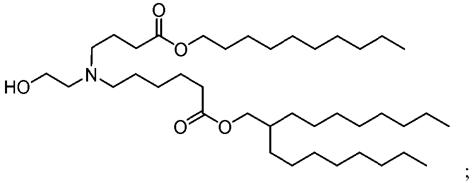 preferably, a molar ratio of the YK-009, 1,2-distearoyl-sn-glycero-3-phosphocholine, cholesterol and 1,2-dimyristoyl-sn-glycero-3-methoxypolyethylene glycol 2000 is 49: 10: 39.5: 1.5.

9. The pharmaceutical composition according to any one of claims 5 to 8, wherein the pharmaceutical composition further comprises one or at least two cell-penetrating peptides.

10. A method for synthesizing mRNA molecules for non-diagnostic and non-therapeutic purpose with respect to diseases, wherein the method comprises the following steps: co-incubating the compound represented by formula (I), a stereoisomer thereof, a pharmaceutically acceptable salt thereof or a solvate thereof according to any one of claim 1 or 2 with a polynucleotide template, and performing transcription of the polynucleotide template.

11. A capping mRNA transcription response system for non-diagnostic and non-therapeutic purpose with respect to diseases, comprising: (1) the compound represented by formula (I), a stereoisomer thereof or a pharmaceutically acceptable salt thereof according to any one of claim 1 or 2; and (2) a polynucleotide template, NTPs, and RNA polymerase.

12. A kit, comprising: (1) the compound represented by formula (I), a stereoisomer thereof or a pharmaceutically acceptable salt thereof according to any one of claim 1 or 2; and (2) nucleoside triphosphate molecules and RNA polymerase; further comprising any one or a combination of at least two of the group consisting of an RNase inhibitor, an inorganic pyrophosphatase, Mg²⁺, a crowding agent, and a buffer.

13. A method for enhancing RNA stability, wherein the method comprises incorporating the compound represented by formula (I), a stereoisomer thereof or a pharmaceutically acceptable salt thereof according to any one of claim 1 or 2 into the RNA.

14. A method for introducing RNA into cells, wherein the method comprises contacting the cells with the RNA molecule according to claim 4 or the pharmaceutical composition according to any one of claims 5 to 8.

15. Use of the RNA molecule according to claim 4 or the pharmaceutical composition according to any one of claims 5 to 8 in the preparation of a vaccine.
